# EUROPEAN PATENT APPLICATION

(11) **EP 4 790 067 A1**
(43) Date of publication of application: **12.08.2026**
(21) Application number: 24874997.0
(22) Date of filing: 04.10.2024
(51) Int. Cl.: C07K 14/705, C12N 5/00

(54) **CHIMERIC ADAPTER PROTEIN COMPRISING O6-BENZYLGUANINE BINDING PROTEIN, AND EXTRACELLULAR VESICLE COMPRISING SAME**

(30) Priority: 04.10.2023 KR 20230131984
(71) Applicant: Daegu Gyeongbuk Institute of Science and Technology, Daegu 42988 (KR)
(72) Inventor: YEA, Kyung Moo, Dalseong-gun, Daegu 43016 (KR); SEO, Dae Ha, Busan 48092 (KR); SHIN, Ji Won, Busan 46532 (KR); JANG, Juhee, Gimhae-si, Gyeongsangnam-do 50828 (KR)
(74) Representative: Caspary, Karsten
(86) International application number: PCT/KR2024/015111
(87) International publication number: WO 2025/075441

(57) **Abstract**

The present disclosure relates to a novel chimeric adaptor protein and an extracellular vesicle including the same. The chimeric adaptor protein according to an aspect is abundantly expressed on the surface of an extracellular vesicle, exhibits excellent conjugation efficiency, and possesses superior bioavailability and modularity, including not interacting with other components on the surface of the extracellular vesicle and preserving the integrity thereof. The extracellular vesicle can be effectively utilized as an efficient nanocarrier when a drug is loaded therein.

## Description

### Technical Field

The present disclosure relates to a novel chimeric adaptor protein and extracellular vesicle including the same.

### Background Art

Extracellular vesicles (EVs) are nano-sized membrane-bound structures. Due to their lower toxicity and reduced immunogenicity compared to other nanoparticles or small molecules, EVs have been actively researched for their potential as drug carriers. Surface engineering, by altering the activity of surface proteins, enables improved targeted delivery efficiency and enhanced interactions with specific cell types or tissues.

Surface engineering of extracellular vesicles is broadly classified into genetic techniques and chemical techniques. Genetic techniques require repetitive genetic manipulation of cells and are time-consuming, which can decrease engineering efficiency. Additionally, the range of molecules that can be engineered on the surface is limited to protein molecules. Among chemical techniques, non-covalent bond-mediated chemical techniques may result in unstable maintenance of surface-engineered molecules in specific environments or over extended periods, because the bonds are inherently temporary and unstable. In contrast, covalent bond-mediated chemical techniques can induce changes in the activity of surface proteins because they directly bind the molecules to be engineered to residues of surface protein molecules of extracellular vesicles. However, this technique is also highly demanding because it requires balancing diversity with the need to minimize steric hindrance, while modifying the surface of the extracellular vesicles without compromising their integrity.

The present inventors have made research efforts to develop a novel surface engineering technology and have developed a chimeric adaptor protein (CAP) having a novel structure capable of conjugation with various functional molecules. Extracellular vesicles expressing CAP of the present disclosure can serve as a universal platform for drug delivery.

### Disclosure of Invention

### Technical Problem

One aspect provides a chimeric adaptor protein including an O6-benzylguanine-binding protein, a transmembrane domain, and a signaling domain.

Another aspect provides a construct including a gene encoding the chimeric adaptor protein, a vector including the construct, or a recombinant cell including the construct or the vector.

Another aspect provides an extracellular vesicle including the chimeric adaptor protein or an extracellular vesicle derived from the recombinant cell.

Another aspect provides the extracellular vesicle, wherein a drug is loaded in the extracellular vesicle.

### Solution to Problem

One aspect provides a chimeric adaptor protein including an O6-benzylguanine-binding protein, a transmembrane domain, and a signaling domain.

As used herein, "chimeric adaptor protein (CAP)" refers to a synthetic complex protein designed to perform a new or improved function by joining domains of different proteins. The chimeric adaptor protein may include a specific protein-binding domain, a functional domain that affects the activity of a pathway, and a linker region connecting each domain. The chimeric adaptor protein of the present disclosure may be bound to an extracellular vesicle and expressed on the surface of the extracellular vesicle. That is, the chimeric adaptor protein of the present disclosure may include a specific protein-binding domain, a transmembrane domain, and a signaling domain. The chimeric adaptor protein of the present disclosure may be expressed on the surface of an extracellular vesicle by binding to the extracellular vesicle, and by binding to a specific protein, may induce signaling within the extracellular vesicle and modulate the activity of the extracellular vesicle.

As used herein, "O6-benzylguanine" refers to a compound in which a benzyl group is bound to guanine and is used as a substrate specifically recognized by a self-labeling enzyme such as SNAP-tag. SNAP-tag can be labeled with a molecule by binding to a benzylguanine derivative. The O6-benzyl guanine is used interchangeably with 6-benzylguanine, O6-benzylguanine, 6-(benzyloxy)-7H-purin-2-amine, O6-benzyl, NSC 637037, 6-O-benzylguanine, 6-benzyl oxyguanine, 6-oxo-benzylguanine, or 4 6-benzyloxyguanine.

As used herein, "self-labeling enzyme" or "self-labeling protein" refers to a protein capable of attaching a specific molecular tag or marker to a target protein by itself. Self-labeling proteins are highly useful tools in the fields of cell biology, molecular biology, and biochemistry because they can attach a specific molecular tag or marker to a target protein without requiring complex chemical reactions or modification procedures. The self-labeling protein may be used interchangeably with terms such as "self-tagging protein" or "autonomously labeling protein". Unless otherwise specified, "self-labeling protein" used without modification is used in a sense that comprehensively includes, without limitation, proteins capable of attaching a molecular tag or marker to an extracellular vesicle.

In an embodiment, the O6-benzylguanine-binding protein may include a self-labeling protein.

In an embodiment, the O6-benzylguanine-binding protein may include a SNAP-tag. The SNAP-tag has a high affinity for O6-benzylguanine.

In an embodiment, the O6-benzylguanine-binding protein may be expressed on the surface of an extracellular vesicle.

In an embodiment, the O6-benzylguanine-binding protein may include the amino acid sequence of SEQ ID NO: 2, a fragment thereof, or an amino acid sequence having at least 70 %, 75 %, 80 %, 85 %, 90 %, 95 %, 96 %, 97 %, 98 %, or 99 % identity thereto.

The chimeric adaptor protein of the present disclosure enables site-specific conjugation with various functional molecules through the O6-benzylguanine-binding protein. For example, the chimeric adaptor protein may form a covalent bond with O6-benzylguanine via the O6-benzylguanine-binding protein, enabling binding to various functional molecules linked to said O6-benzylguanine.

As used herein, "transmembrane domain" refers to a domain that connects an extracellular domain and an intracellular domain and is located in a cell membrane. The transmembrane domain of the present disclosure may serve to anchor, display, or bind an O6-benzylguanine-binding protein to a cell membrane of an extracellular vesicle.

In an embodiment, the transmembrane domain may include any one or more transmembrane domains selected from the group consisting of platelet-derived growth factor receptor (PDGFR), epidermal growth factor receptor (EGFR), fibroblast growth factor receptor (FGFR), vascular endothelial growth factor receptor (VEGFR), hepatocyte growth factor receptor (HGFR), tropomyosin receptor kinase (Trk), insulin receptor (IR), leukocyte receptor tyrosine kinase (LTK), angiopoietin receptor, cholecystokinin (CCK) receptor, neurotrophic factor (NGF) receptor, receptor tyrosine kinase-like orphan receptors (ROR), discoidin domain receptor (DDR), rearranged during transfection receptor (RETR), tyrosine-protein kinase-like (PTK), related to receptor tyrosine kinase (RYK), muscle-specific kinase (MuSK), CD63, CD9, and CD81. Preferably, the transmembrane domain may include a transmembrane domain of platelet-derived growth factor receptor (PDGFR).

In an embodiment, the transmembrane domain may include the amino acid sequence of SEQ ID NO: 4, a fragment thereof, or an amino acid sequence having at least 70 %, 75 %, 80 %, 85 %, 90 %, 95 %, 96 %, 97 %, 98 %, or 99 % identity thereto.

As used herein, "signaling domain" refers to a portion located inside the cell membrane, that is, within the interior of the extracellular vesicle, and refers to a domain that transmits signals transferred by the binding of a ligand or the like to the extracellular domain.

In an embodiment, the signaling domain may include one or more signaling domains selected from the group consisting of CD9, CD63, C-terminus of CD63, CD81, LAMP-1, LAMP-2, syntaxin-3, syntenin-1, syntenin-2, syndecan-1, syndecan-4, and prostaglandin F2 receptor negative regulator. Preferably, the signaling domain may include the N-terminal sorting domain (NTD) of syntenin. The NTD is a signal sequence required for directing a protein to move to a specific organelle or location within a cell and is involved in protein targeting and transport.

The signaling domain in the chimeric adaptor protein of the present disclosure may serve to increase the surface abundance of the chimeric adaptor protein during the biogenesis of the extracellular vesicles.

In an embodiment, the signaling domain may include the amino acid sequence of SEQ ID NO: 6, a fragment thereof, or an amino acid sequence having at least 70 %, 75 %, 80 %, 85 %, 90 %, 95 %, 96 %, 97 %, 98 %, or 99 % identity thereto.

In an embodiment, the O6-benzylguanine-binding protein and the transmembrane domain may be linked directly or via a linker. That is, the linker domain may be located between the O6-benzylguanine-binding protein and the transmembrane domain.

Each domain of the chimeric adaptor protein of the present disclosure may be optionally connected by a short oligopeptide or polypeptide linker. The linker may be a rigid linker or a flexible linker, and its length or type is not particularly limited; any linkers known in the art may be applied without limitation.

In the present disclosure, the linker may preferably include a rigid linker. More specifically, the linker may be (EAAAK)ₙ, wherein the copy number "n" may be adjusted in consideration of the optimization of the linker. For example, the copy number n may be an integer from 5 to 10, and preferably, n may be 8.

In an embodiment, the linker may include the amino acid sequence of SEQ ID NO: 3, a fragment thereof, or an amino acid sequence having at least 70 %, 75 %, 80 %, 85 %, 90 %, 95 %, 96 %, 97 %, 98 %, or 99 % identity thereto.

The linker in the chimeric adaptor protein of the present disclosure may improve the conjugation efficiency between the O6-benzylguanine-binding protein and O6-benzylguanine.

In an embodiment, the chimeric adaptor protein may further include a reporter.

As used herein, "reporter" is used to monitor whether the self-labeling protein of the present disclosure is incorporated or to monitor expression efficiency, and any protein that can be monitored non-destructively may be used without limitation.

In an embodiment, the reporter may be luciferase, green fluorescent protein (GFP), modified green fluorescent protein (mGFP), enhanced green fluorescent protein (EGFP), red fluorescent protein (RFP), modified red fluorescent protein (mRFP), enhanced red fluorescent protein (ERFP), blue fluorescent protein (BFP), enhanced blue fluorescent protein (EBFP), yellow fluorescent protein (YFP), enhanced yellow fluorescent protein (EYFP), cyan fluorescent protein (CFP), or enhanced cyan fluorescent protein (ECFP).

In an embodiment, the reporter may be located between the transmembrane domain and the signaling domain.

In an embodiment, the reporter may include the amino acid sequence of SEQ ID NO: 5, a fragment thereof, or an amino acid sequence having at least 70 %, 75 %, 80 %, 85 %, 90 %, 95 %, 96 %, 97 %, 98 %, or 99 % identity thereto.

The chimeric adaptor protein of the present disclosure is expressed abundantly on the surface of extracellular vesicles as described above, exhibits excellent conjugation efficiency with O6-benzylguanine, and minimizes interference with other cell membrane components, such as neighboring proteins. The chimeric adaptor protein of the present disclosure strategically incorporates a long rigid linker and syntenin NTD to minimize steric hindrance of the local environment on the surface of extracellular vesicles, thereby enabling efficient binding with target molecules and providing enhanced functions. In addition, the extracellular vesicle into which the chimeric adaptor protein of the present disclosure is introduced preserves its integrity as an isolated, distinct entity. That is, the chimeric adaptor protein of the present disclosure can impart modularity without disrupting the surrounding environment on the surface of the extracellular vesicle.

In an embodiment, the chimeric adaptor protein may include the amino acid sequence of SEQ ID NO: 1, a fragment thereof, or an amino acid sequence having at least 70 %, 75 %, 80 %, 85 %, 90 %, 95 %, 96 %, 97 %, 98 %, or 99 % identity thereto.

As used herein, "homology" and "identity" refer to the degree of relation between two given sequences, expressed as a percentage. The terms homology and identity may often be used interchangeably. Sequence homology or identity may be determined using a known computer algorithm such as the 'FASTA' program using default parameters (Pearson et al., Proc. Natl. Acad. Sci. USA 85: 2444, 1988). Alternatively, the Needleman-Wunsch algorithm (Needleman and Wunsch, J. Mol. Biol. 48: 443-453, 1970) may be used for the determination, as performed in the Needle program of the EMBOSS package (Rice et al., Trends Genet. 16: 276-277, 2000) (version 5.0.0 or later), or using BLASTP, BLASTN, or FASTA (Altschul et al., J. Mol. Biol. 215: 403, 1990; Carillo et al., SIAM J. Applied Math. 48: 1073, 1988). For example, sequence homology or identity may be determined using BLAST from the National Center for Biotechnology Information or ClustalW.

Each domain of the chimeric adaptor protein described above in the present specification may include the aforementioned domains as well as modified forms thereof. In this case, the modifications may be performed by substituting, deleting, or adding one or more amino acids in the amino acid sequence of the wild-type domain without altering the functions of the domains. Typically, the substitution may be performed by a conservative amino acid substitution that does not affect the charge, polarity, or hydrophobicity of the entire protein.

Another aspect provides a construct including a gene encoding the chimeric adaptor protein.

Another aspect provides a vector including the construct.

As used herein, "construct" refers to a macromolecule or molecular complex including a polynucleotide delivered to a host cell in any one of *in vitro, in vivo*, or *ex vivo*. The construct may further include a promoter sequence.

As used herein, the term "encoding" refers to the inherent property of a specific sequence of nucleotides in a polynucleotide, such as a gene, cDNA, or mRNA, to serve as a template for the synthesis of other polymers and macromolecules in a biological process having either a defined sequence of nucleotides (i.e., rRNA, tRNA, and mRNA) or a defined sequence of amino acids, and the biological properties resulting therefrom. As such, a gene encodes a protein if the transcription and translation of mRNA corresponding to that gene in a cell or other biological system produces the protein. Both the coding strand, which is identical to the mRNA sequence and is the nucleotide sequence usually provided in a sequence listing, and the non-coding strand, which is used as a template for transcription of a gene or cDNA, can be said to encode the protein or other product of that gene or cDNA.

As used herein, "vector" refers to any nucleic acid construct capable of delivering foreign genetic material to a target cell in which the polynucleotide can be replicated and/or expressed, or capable of directing transport. The vector includes a construct to be delivered. The vector may be a linear molecule or a circular molecule. The vector may be an integrating or non-integrating vector.

In an embodiment, the vector may be any one selected from the group consisting of a plasmid, a cosmid, a virus, a phage, a recombinant expression cassette, and a transposon. However, the vector is not limited thereto, and it will be understood that any suitable vector known in the art may be used.

It will be understood that a gene sequence in which a portion of the sequence is deleted, modified, substituted, or added from the construct or the vector of the present disclosure may also be used in the present application, provided that it has a function identical or corresponding to that of the gene of the present disclosure.

In an embodiment, the construct may include the gene sequence of SEQ ID NO: 7, a fragment thereof, or a gene sequence having at least 70 %, 75 %, 80 %, 85 %, 90 %, 95 %, 96 %, 97 %, 98 %, or 99 % identity thereto.

In an embodiment, the construct may include any one or more gene sequences of SEQ ID NOs: 8 to 12, a fragment thereof, or a gene sequence having at least 70 %, 75 %, 80 %, 85 %, 90 %, 95 %, 96 %, 97 %, 98 %, or 99 % identity thereto.

Another aspect provides a recombinant cell including the construct or the vector.

The recombinant cell may include a single vector containing a single gene encoding the chimeric adaptor protein. The recombinant cell may be one into which a single vector containing genes encoding the domains of the chimeric adaptor protein has been introduced, or one into which a plurality of vectors, each containing the respective genes, have been introduced. Each gene or vector may be independently introduced into the recombinant cell simultaneously, sequentially, or in reverse order.

In an embodiment, the recombinant cell may be an archaeal cell, a bacterial cell, a eukaryotic cell, a eukaryotic unicellular organism, a somatic cell, a germ cell, a stem cell, a plant cell, an animal cell, a mammalian cell, a mouse cell, a non-human primate cell, or a human cell.

Another aspect provides an extracellular vesicle including the chimeric adaptor protein, or an extracellular vesicle derived from the recombinant cell.

As used herein, "extracellular vesicle (EV)" refers to any and all types of vesicles that are nano-sized, membrane-enclosed structures originating from the endosomal compartments of eukaryotic cells and released into the extracellular space. "Extracellular vesicle," "cell-derived vesicle," and "vesicles or sacs released to the extracellular environment" are used interchangeably, and the extracellular vesicle may include various subpopulations such as exosomes, ectosomes, microvesicles, microparticles, and exosome-like vesicles. Small extracellular vesicle (sEV) generally refers to an extracellular vesicle having a diameter of 50 to 200 nm, and the extracellular vesicle of the present disclosure may be a small extracellular vesicle, but is not limited thereto.

Extracellular vesicles engineered with the chimeric adaptor protein of the present disclosure may be used as powerful vehicles for targeted drug delivery based on the excellent bioavailability and pharmacokinetic properties inherent to extracellular vesicles themselves.

In an embodiment, the extracellular vesicle may have a diameter of 0.1 to 1,000 nm. Specifically, the extracellular vesicle may have a diameter of 50 to 200 nm.

In an embodiment, the extracellular vesicle may have O6-benzylguanine, or O6-benzylguanine and a functional molecule additionally linked to the chimeric adaptor protein.

As used herein, "functional molecule" refers to any molecule, without limitation, capable of binding with the extracellular vesicle of the present disclosure by binding with O6-benzylguanine. Extracellular vesicles engineered with the chimeric adaptor protein of the present disclosure exhibit excellent engineering efficiency with the functional molecule.

Specifically, the functional molecule may be an organic fluorophore, an inorganic material, an antibody or an antigen-binding fragment thereof, or a cytokine, but is not limited thereto.

In an embodiment, the organic fluorophore may be SNAP 549, Alexa Fluor 488, Alexa Fluor 546, Alexa Fluor 647, Cy3, Cy5, Cy7, FITC, Rhodamine B, Texas Red, DAPI, fluorescein, Atto 488, Atto 550, or Atto 647, but is not limited thereto.

In an embodiment, the inorganic material may be quantum dots (QD) or a QD conjugate, or other nanoparticles (NP) or an NP conjugate. For example, the inorganic material may be Streptavidin-QD, CdSe/ZnS-QD, CdTe-QD, PbS-QD, InP-QD, ZnS-QD, ZnO NP, BaTiO₃ NP, Fe₃O₄ NP, CuO NP, Cu₂O NP, Au NP, Streptavidin-Au NP, or Ag NP, but is not limited thereto.

In an embodiment, the antibody or an antigen-binding fragment thereof may include any one or more selected from the group consisting of monoclonal antibody, domain antibody (dAb), single chain antibody (scAb), Fab fragment, Fab' fragment, F(ab')₂ fragment, scFab fragment, Fv fragment, dsFv fragment, single chain variable fragment (scFv), scFv-Fc fragment, single domain heavy chain antibody, single domain light chain antibody, variant antibody, multimeric antibody, minibody, diabody, bispecific antibody, and multispecific antibody.

In an embodiment, the antibody or an antigen-binding fragment thereof may be an anti-EGFR antibody. More specifically, the antibody or an antigen-binding fragment thereof may be cetuximab, panitumumab, or necitumumab, but is not limited thereto.

In an embodiment, the cytokine may be IL-2, IL-7, IL-12, IL-15, IL-21, IFN-α, IFN-γ, TNF-α, TGF-β, IL-10, IL-1β, IL-6, IL-17, or GM-CSF, but is not limited thereto.

In an embodiment, a drug may be loaded in the extracellular vesicle. That is, the extracellular vesicle of the present disclosure may function as a drug delivery vehicle and may exhibit preventive, ameliorative, or therapeutic effects depending on the type of drug incorporated therein. For example, a tumor-targeting therapeutic agent, when associated with the extracellular vesicle, may exhibit preventive, ameliorative, or therapeutic effects against various tumors.

The drug may be included without limitation as long as it is a drug that can be loaded inside the extracellular vesicle. For example, the drug may be an RNA-based drug (mRNA, siRNA, miRNA, etc.), a therapeutic protein, an anticancer agent, an antibody, a cytokine, a gene editing tool, or a lipid-based drug, but is not limited thereto.

Specifically, the anticancer agent may include drugs such as doxorubicin, cyclophosphamide, paclitaxel, carboplatin, cisplatin, methotrexate, ifosfamide, topotecan, vincristine, or tamoxifen. Preferably, the anticancer agent may be doxorubicin.

In addition, examples of the antibody may include alemtuzumab, apolizumab, aselizumab, atlizumab, bapineuzumab, bevacizumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pertuzumab, pexelizumab, ralivizumab, ranibizumab, reslivizumab, reslizumab, resavizumab, rovelizumab, ruplizumab, sibrotuzumab, siplizumab, sontuzumab, tacatuzumab tetraxetan, tadocizumab, talizumab, tepivazumab, tocilizumab, toralizumab, trastuzumab, tucotuzumab celmoleukin, tucusituzumab, umavizumab, urtoxazumab, or visilizumab.

While the present disclosure is capable of various modifications and alternative embodiments, specific embodiments thereof are shown by way of example in the drawings and will herein be described in detail. It should be understood, however, that there is no intent to limit the disclosure to the particular forms disclosed, but on the contrary, the disclosure is to cover all modifications, equivalents, and alternatives falling within the spirit and scope of the disclosure. In the description, descriptions of related well-known art may be omitted where they would obscure the disclosure with unnecessary detail.

### Advantageous Effects of Invention

The chimeric adaptor protein according to an aspect is abundantly expressed on the surface of an extracellular vesicle, exhibits excellent conjugation efficiency, and possesses superior bioavailability and modularity, including not interacting with other components on the surface of the extracellular vesicle and preserving the integrity thereof. The extracellular vesicle can be effectively utilized as an efficient nanocarrier when a drug is loaded therein.

### Brief Description of Drawings

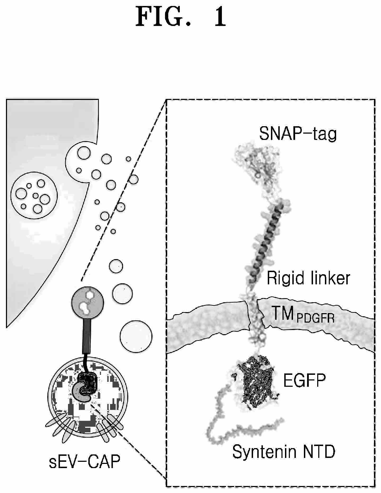

FIG. 1 is a structural design diagram of an extracellular vesicle expressing a chimeric adaptor protein (sEV-CAP) according to an embodiment.
FIG. 2 shows a TEM image of sEV-CAP and a graph showing the diameter and size distribution thereof according to an embodiment.
FIG. 3 is a graph comparing production yields of sEV-CAP according to an embodiment.
FIG. 4 is an image showing western blotting results of sEV-CAP according to an embodiment.
FIG. 5 is an image showing CAP expression levels in HEK293FT cells according to the presence or absence of syntenin NTD, based on the comparative analysis of EGFP fluorescence intensity linked to the intracellular domain of the chimeric adaptor protein.
FIG. 6 is an image quantifying CAP expression levels in a single extracellular vesicle based on EGFP counts in the presence or absence of syntenin NTD.
FIG. 7 is a TIRF image showing EGFP and labeled QD in a cell expressing CAP and a graph quantifying QD labeling yield according to an embodiment.
FIG. 8 is an image showing the trajectories of CAP-conjugated QDs in HEK293FT cells expressing CAP.
FIG. 9 is a schematic diagram illustrating the modular engineering of sEV-CAP combined with various functional molecules.
FIG. 10 shows TIRF images and correlation coefficients showing O6-benzylguanine (BG)-dependent colocalization between sEV-CAP and various functional molecules according to an embodiment.
FIG. 11 presents images showing the distribution of CAPs and functional molecules in sEV-CAP bound to functional molecules.
FIG. 12 is a TEM image of sEV-CAP bound with QD (sEV-QD).
FIG. 13 is a graph illustrating a comparison between the average number of QDs bound to sEV-CAP analyzed through TEM and the results of stoichiometric analysis of sEV-QD based on TEM and optical microscopy (OM).
FIG. 14 presents graphs showing linear regression analysis results for each CAP and functional molecule in sEV-CAPs bound to functional molecules.
FIG. 15 is a schematic diagram illustrating the surface engineering and activity assays of sEV-CAP.
FIG. 16 shows graphs illustrating SEAP activity induced by sEV-IL2 and dose-response curves for sEVs.
FIG. 17 shows time-lapse EGFP fluorescence images of a single cell after treatment with sEV-Y.
FIG. 18 shows graphs illustrating EGFP fluorescence intensity in a single cell over time after treatment with sEV-Y (left) and EGFP fluorescence intensity on a single-cell basis one hour after treatment with sEV-Y (right).
FIG. 19 is a graph showing mean square displacement (MSD) analysis results for trajectories exhibiting directed motion two hours after treatment with sEV-Y.
FIG. 20 is a schematic diagram illustrating drug delivery by sEV(drug)-Y according to an embodiment.
FIG. 21 is a TIRF image illustrating the loading of doxorubicin (Dox) into sEV-Y to generate sEV(Dox)-Y as shown by colocalization of sEV-Y and Dox fluorescence.
FIG. 22 shows a fluorescence image and a graph illustrating the quantification of intracellular delivery after treatment with sEV(Dox)-Y.
FIG. 23 is a graph illustrating cell viability after treatment with sEV(Dox)-Y.
FIG. 24 is a schematic diagram illustrating a drug administration schedule in mice for tumor targeting analysis of sEV-Y according to an embodiment.
FIG. 25 shows ex vivo fluorescence images in tumors and major organs after administration of wt sEV, sEV, and sEV-Y.
FIG. 26 is a graph illustrating the quantification of DiR intensity within tumors after administration of wt sEV, sEV, and sEV-Y.
FIG. 27 shows images illustrating the accumulation of DiR-labeled sEV in frozen tumor sections after administration of wt sEV, sEV, and sEV-Y.
FIG. 28 is a schematic diagram illustrating the administration schedule in mice for a drug delivery efficacy assay of sEV-Y according to an embodiment.
FIG. 29 is a graph illustrating the quantification of tumor bioluminescence measured in mice on the day of euthanasia.
FIG. 30 shows photographs of tumor tissue and a graph illustrating tumor weights obtained from mice on the day of euthanasia.
FIG. 31 illustrates tumor volume curves over 19 days from the initial day of treatment in mice.
FIG. 32 illustrates body weight change curves over 19 days from the initial day of treatment in mice.
FIG. 33 shows immunostaining images and a graph illustrating the quantification of TUNEL signals in tumor tissue after drug treatment in mice, expressed as a fold change.
FIG. 34 shows histological images of tumor tissue stained with hematoxylin and eosin after drug treatment in mice.

### Mode for the Invention

Hereinafter, preferred embodiments of the present disclosure will be described to facilitate understanding of the disclosure. However, the following examples are provided only for easier understanding of the present disclosure, and the scope of the present disclosure is not limited by the following examples. The embodiments may be implemented in various forms and are not limited to the embodiments disclosed herein, as various modifications may be made thereto.

### Example 1. Preparation of sEV-CAP

### 1.1 Introduction of the CAP

Extracellular vesicles (sEV-CAP) expressing a chimeric adaptor protein (CAP) according to an embodiment were prepared as follows.

A vector encoding the CAP was constructed by synthesizing a gene encoding the CAP and then introducing the synthesized gene into a pLV2 vector. After transfecting the vector encoding the CAP into HEK293FT cells using Lipofectamine 2000, the transfected cells were cultured in a culture medium supplemented with the antibiotic hygromycin (300 µg ml⁻¹) for 21 days.

The gene and amino acid sequences of the CAP are shown in Tables 1 and 2 below.

**[Table 1]**

| **Type** | **Amino Acid Sequence** |
|---|---|
| Full-length CAP sequence (SEQ ID NO: 1) | MDKDCEMKRTTLDSPLGKLELSGCEQGLHRIIFLGKGTSAAD AVEVPAPAAVLGGPEPLMQATAWLNAYFHQPEAIEEFPVPAL HHPVFQQESFTRQVLWKLLKVVKFGEVISYSHLAALAGNPAA TAAVKTALSGNPVPILIPCHRVVQGDLDVGGYEGGLAVKEWL LAHEGHRLGKPGLGGSEAAAKEAAAKEAAAKEAAAKEAAAK EAAAKEAAAKEAAAKGSTSGSGKPGSGEGSTKGGSAVGQD TQEVIVVPHSLPFKVVVISAILALVVLTIISLIILIMLWQKKPRGG GGSMVSKGEELFTGVVPILVELDGDVNGHKFSVSGEGEGDA TYGKLTLKFICTTGKLPVPWPTLVTTLTYGVQCFSRYPDHMK QHDFFKSAMPEGYVQERTIFFKDDGNYKTRAEVKFEGDTLV NRIELKGIDFKEDGNILGHKLEYNYNSHNVYIMADKQKNGIKV NFKIRHNIEDGSVQLADHYQQNTPIGDGPVLLPDNHYLSTQS ALSKDPNEKRDHMVLLEFVTAAGITLGMDELYKGGGGSSLYP SLEDLKVDKVIQAQTAFSANPANPAILSEASAPIPHDGNLYPR LYPELSQYMGLSLEGG |
| SNAP-tag (SEQ ID NO: 2) | MDKDCEMKRTTLDSPLGKLELSGCEQGLHRIIFLGKGTSAAD AVEVPAPAAVLGGPEPLMQATAWLNAYFHQPEAIEEFPVPAL HHPVFQQESFTRQVLWKLLKVVKFGEVISYSHLAALAGNPAA TAAVKTALSGNPVPILIPCHRVVQGDLDVGGYEGGLAVKEWL LAHEGHRLGKPGLG |
| Rigid linker (SEQ ID NO: 3) | EAAAKEAAAKEAAAKEAAAKEAAAKEAAAKEAAAKEAAAK |
| PDGFR beta TM (SEQ ID NO: 4) | AVGQDTQEVIVVPHSLPFKVVVISAILALVVLTIISLIILIMLWQK KPR |
| EGFP (SEQ ID NO: 5) | MVSKGEELFTGVVPILVELDGDVNGHKFSVSGEGEGDATYG KLTLKFICTTGKLPVPWPTLVTTLTYGVQCFSRYPDHMKQHD FFKSAMPEGYVQERTIFFKDDGNYKTRAEVKFEGDTLVNRIE LKGIDFKEDGNILGHKLEYNYNSHNVYIMADKQKNGIKVNFKI RHNIEDGSVQLADHYQQNTPIGDGPVLLPDNHYLSTQSALSK DPNEKRDHMVLLEFVTAAGITLGMDELYK |
| Syntenin NTD (SEQ ID NO: 6) | SLYPSLEDLKVDKVIQAQTAFSANPANPAILSEASAPIPHDGN LYPRLYPELSQYMGLSLEGG |

**[Table 2]**

| **Type** | **Gene Sequence** |
|---|---|
| Full-length CAP sequence (SEQ ID NO: 7) | tgaccctgaagttcatctgcaccaccggcaagctgcccgtgccctggcccaccctcgt gaccaccctgacctacggcgtgcagtgcttcagccgctaccccgaccacatgaagc agcacgacttcttcaagtccgccatgcccgaaggctacgtccaggagcgcaccatctt cttcaaggacgacggcaactacaagacccgcgccgaggtgaagttcgagggcgac accctggtgaaccgcatcgagctgaagggcatcgacttcaaggaggacggcaaca tcctggggcacaagctggagtacaactacaacagccacaacgtctatatcatggccg acaagcagaagaacggcatcaaggtgaacttcaagatccgccacaacatcgagg acggcagcgtgcagctcgccgaccactaccagcagaacacccccatcggcgacg gccccgtgctgctgcccgacaaccactacctgagcacccagtccgccctgagcaaa gaccccaacgagaagcgcgatcacatggtcctgctggagttcgtgaccgccgccgg gatcactctcggcatggacgagctgtacaagggcggtggcggttcctctctctatccat ctctcgaagacttgaaggtagacaaagtaattcaggctcaaactgctttttctgcaaac cctgccaatccagcaattttgtcagaagcttctgctcctatccctcacgatggaaatctct atcccagactgtatccagagctctctcaatacatggggctgagtttagaaggcggt |
| SNAP-tag (SEQ ID NO: 8) | atggacaaagactgcgaaatgaagcgcaccaccctggatagccctctgggcaagct ggaactgtctgggtgcgaacagggcctgcaccgtatcatcttcctgggcaaaggaac atctgccgccgacgccgtggaagtgcctgccccagccgccgtgctgggcggaccag agccactgatgcaggccaccgcctggctcaacgcctactttcaccagcctgaggcca tcgaggagttccctgtgccagccctgcaccacccagtgttccagcaggagagctttac ccgccaggtgctgtggaaactgctgaaagtggtgaagttcggagaggtcatcagcta cagccacctggccgccctggccggcaatcccgccgccaccgccgccgtgaaaacc gccctgagcggaaatcccgtgcccattctgatcccctgccaccgggtggtgcagggc gacctggacgtggggggctacgagggcgggctcgccgtgaaagagtggctgctgg cccacgagggccacagactgggcaagcctgggctgggt |
| Rigid linker (SEQ ID NO: 9) | gaagccgctgctaaggaagccgctgctaaggaagccgctgctaaggaagccgctg ctaaggaagccgctgctaaggaagccgctgctaaggaagccgctgctaaggaagc cgctgctaag |
| PDGFR beta TM (SEQ ID NO: 10) | gctgtgggccaggacacgcaggaggtcatcgtggtgccacactccttgccctttaagg tggtggtgatctcagccatcctggccctggtggtgctcaccatcatctcccttatcatcctc atcatgctttggcagaagaagccacgt |
| EGFP (SEQ ID NO: 11) | atggtgagcaagggcgaggagctgttcaccggggtggtgcccatcctggtcgagctg gacggcgacgtaaacggccacaagttcagcgtgtccggcgagggcgagggcgat gccacctacggcaagctgaccctgaagttcatctgcaccaccggcaagctgcccgtg ccctggcccaccctcgtgaccaccctgacctacggcgtgcagtgcttcagccgctacc ccgaccacatgaagcagcacgacttcttcaagtccgccatgcccgaaggctacgtcc aggagcgcaccatcttcttcaaggacgacggcaactacaagacccgcgccgaggt gaagttcgagggcgacaccctggtgaaccgcatcgagctgaagggcatcgacttca aggaggacggcaacatcctggggcacaagctggagtacaactacaacagccaca acgtctatatcatggccgacaagcagaagaacggcatcaaggtgaacttcaagatc cgccacaacatcgaggacggcagcgtgcagctcgccgaccactaccagcagaac acccccatcggcgacggccccgtgctgctgcccgacaaccactacctgagcaccca gtccgccctgagcaaagaccccaacgagaagcgcgatcacatggtcctgctggagt tcgtgaccgccgccgggatcactctcggcatggacgagctgtacaag |
| Syntenin NTD (SEQ ID NO: 12) | tctctctatccatctctcgaagacttgaaggtagacaaagtaattcaggctcaaactgctt tttctgcaaaccctgccaatccagcaattttgtcagaagcttctgctcctatccctcacga tggaaatctctatcccagactgtatccagagctctctcaatacatggggctgagtttaga aggcggt |

### 1.2 Isolation of sEV-CAP

sEVs were isolated from the culture supernatant of the cells stably expressing the chimeric adaptor protein (CAP) as described in Example 1.

First, the cells were allowed to stabilize for 16 hours after seeding. Then, the cells were washed three times with PBS and then cultured for 24 hours in serum-free DMEM supplemented with 1 % A/A, insulin (1 mg ml⁻¹), transferrin (550 µg ml⁻¹), and sodium selenite (670 ng ml⁻¹). The culture supernatant was collected and subjected to serial centrifugation at 300 g for 5 minutes and at 2,500 g for 20 minutes, followed by filtration through a 0.22 µm filter. The sEVs were purified through a tangential flow filtration (TFF) system using a hollow fiber filter (polysulfone MidiKros TFF device with a 0.05 µm pore size, Spectrum Laboratories). The filtered supernatant was continuously circulated while maintaining a pressure of 20 psi or less, which was monitored by an inline analog pressure gauge. Then, the sEVs were concentrated after diafiltration against PBS. The sEVs were stained with DiR according to the manufacturer's instructions, and free DiR was removed against PBS using the TFF system.

### Example 2. Surface Engineering of sEV-CAP

Surface engineering of the extracellular vesicles expressing the chimeric adaptor protein (sEV-CAP) prepared in Example 1 with various functional molecules was performed as follows.

Specifically, sEV-CAP and BG-linker-conjugated molecules were mixed and reacted at room temperature in PBS under rotation overnight. For organic dye conjugation, 1.0 µM SNAP 549 was reacted with sEV-CAP. For inorganic nanomaterial (QD, Au NP) conjugation, sEV-CAP was incubated with 1 µM BG-biotin and washed, and then reacted with 1.0 nM streptavidin-QD or 0.1 nM streptavidin-Au NP. For protein conjugation (antibody), cetuximab was conjugated with RPE at four N-glycan domains of the Fc region using a SiteClick^{™} antibody labeling kit (Invitrogen) according to the manufacturer's instructions. Then, 20.0 µM RPE-conjugated cetuximab (Cet-RPE) was mixed and reacted with a 20-fold molar excess of BG-GLA-NHS. Simultaneously, 20 µM IL2 was pre-reacted with a 5-fold molar excess of BG-GLA-NHS at room temperature for 30 minutes. Then, unconjugated BG-GLA-NHS was removed from each mixture using a 7 K MWCO Zeba spin desalting column (Thermo Fisher). Finally, 1.0 nM BG-conjugated Cet-RPE or 1.0 nM BG-conjugated IL2 was reacted with sEV-CAP. After the reaction, the sEV-CAP conjugated with each molecule was washed using the TFF system to remove unconjugated molecules, except for sEV-Au NP. For control experiments, NH₂-Tex, streptavidin-QD, and Cet-RPE without a BG-linker were also reacted with sEV-CAP in PBS at room temperature overnight under rotation. After incubation, sEV-CAP and the control samples were washed using the TFF system to remove unconjugated functional molecules.

### Experimental Example 1. Evaluation of Extracellular Vesicle Characteristics of sEV-CAP

Characteristics of extracellular vesicles expressing the chimeric adaptor protein (sEV-CAP) according to an embodiment were evaluated as follows.

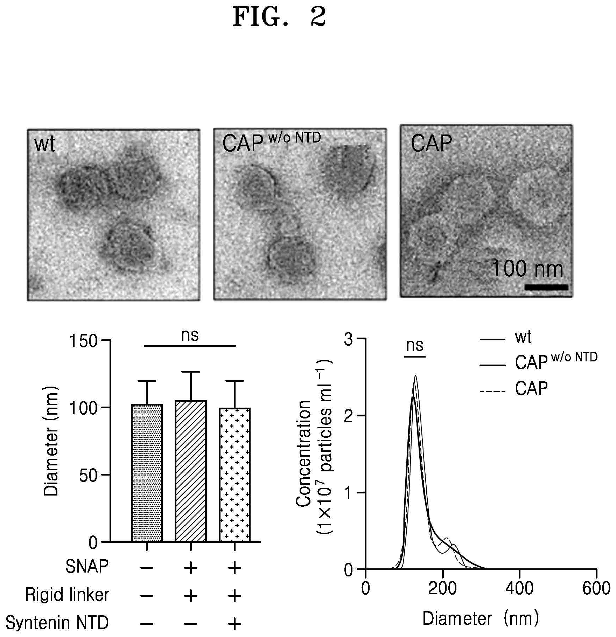

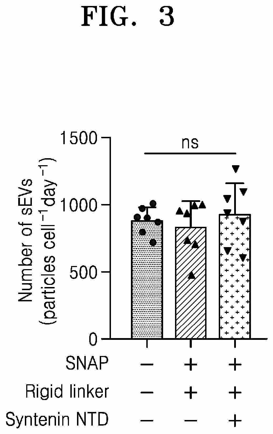

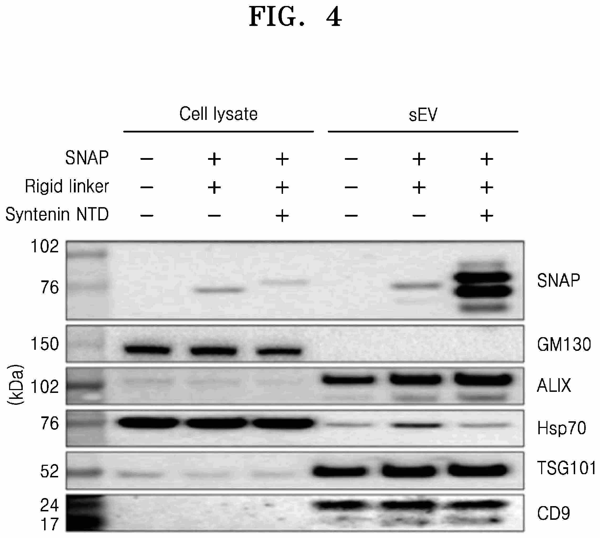

First, it was investigated whether sEV-CAP affected the intrinsic characteristics of extracellular vesicles. Specifically, after harvesting and purifying sEVs from HEK293FT cells using tangential flow filtration (TFF), sEVs expressing CAP (sEV-CAP), sEVs expressing CAP lacking the syntenin NTD (sEV-CAP^{w/o NTD}), and wild-type sEVs (wt sEV) were comparatively analyzed. Size and morphology were compared using transmission electron microscopy (TEM), as shown in FIG. 2. Biosynthesis efficiency was compared using nanoparticle tracking analysis (NTA), as shown in FIG. 3. sEV markers were identified using Western blotting, as shown in FIG. 4.

As shown in FIG. 2, the diameter and size distribution were similar among the tested groups of extracellular vesicles.

As shown in FIG. 3, the production yields were found to be wt sEV (710 ± 73 cells⁻¹ day⁻¹), sEV-CAP^{w/o NTD} (668 ± 145 cells⁻¹ day⁻¹), and sEV-CAP (743 ± 177 cells⁻¹ day⁻¹).

As shown in FIG. 4, the presence of typical sEV markers (ALIX, Hsp70, TSG101, and CD9), the absence of GM130 (a Golgi marker which is a non-sEV marker), and the co-expression of SNAP-tag were confirmed in all extracellular vesicle groups.

That is, there was no significant difference in size, morphology, and biosynthesis efficiency among sEV-CAP, sEV-CAP^{w/o NTD}, and wt sEV prepared according to an embodiment.

### Experimental Example 2. Evaluation of Efficacy of sEV-CAP

Biophysical characteristics of the CAP structure in sEV-CAP, which contribute to efficient surface engineering of extracellular vesicles, were evaluated in the following experiments.

### 2.1 Verification of CAP Localization

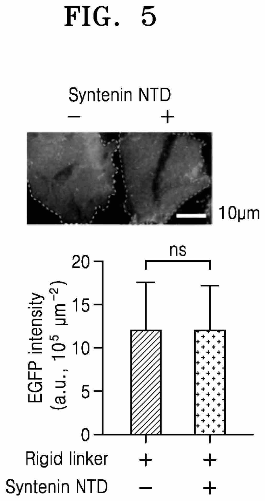

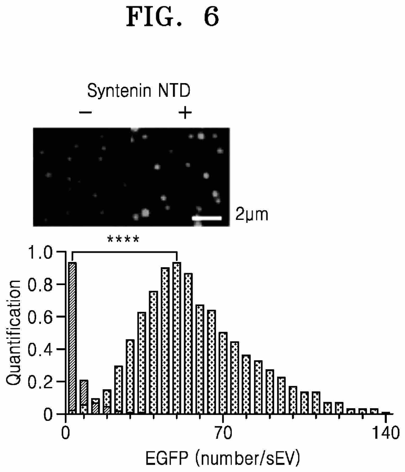

Using total internal reflection fluorescence (TIRF) microscopy, the EGFP fluorescence intensity of CAP was measured in HEK293FT cells in the presence or absence of the syntenin NTD. Results of the comparative analysis of CAP expression levels at the cellular level are shown in FIG. 5. In addition, the fluorescence intensity of EGFP (I_{EGFP}) in sEVs expressing CAP (sEV-CAP) and sEVs expressing CAP without syntenin NTD (sEV-CAP^{w/o NTD}) was measured at a single-molecule level. The content of individual sEVs was then quantified by dividing by I_{EGFP}, with the results shown in FIG. 6.

As shown in FIG. 5, the syntenin NTD did not alter the CAP expression levels in the cells. Meanwhile, as shown in FIG. 6, syntenin NTD significantly improved CAP localization in sEVs, increasing the number of units per sEV from 8 ± 6 (sEV-CAP^{w/o NTD}) to 59 ± 24 (sEV-CAP).

### 2.2 Measurement of Macromolecule Labeling Efficiency

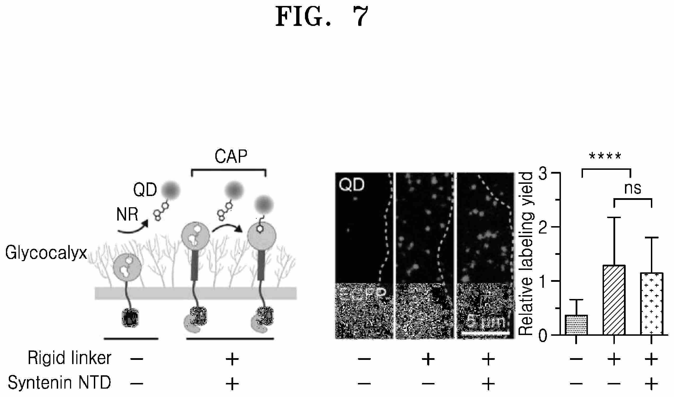

Additionally, to evaluate the binding efficiency of CAP and macromolecules such as antibodies, QD labeling efficiency was measured in HEK293FT cells. Specifically, BG-conjugated quantum dots (QD) were used in a sequential treatment involving BG-biotin, followed by streptavidin-QD. QDs enable quantitative analysis through optical signals and have a size (approximately 15 nm) similar to that of antibodies; thus, QDs are suitable for evaluating potential steric constraints during protein functionalization. Results of comparative analysis of HEK293FT cells expressing CAP or CAP lacking the syntenin NTD (CAP^{w/o NTD}) and wild-type HEK293FT cells are shown in FIG. 7.

As shown in FIG. 7, for wild-type (wt) HEK293FT cells lacking the rigid linker and syntenin NTD, binding to BG significantly decreased and was indicated as non-reactive (NR). As can be seen from TIRF images illustrating labeled QD and EGFP in HEK293FT cells expressing each variant (CAP or CAP^{w/o NTD}) and the graph quantifying labeling yield, binding with BG increased upon binding of the rigid linker, and binding efficiency was not impaired even when syntenin NTD was added.

### 2.3 Measurement of Interaction with Neighboring Proteins

Since membrane receptors can be activated or inhibited through interactions, whether CAP physically engages in protein-protein interactions (PPI) with other proteins or organelles was investigated to evaluate potential functional disturbances of neighboring proteins.

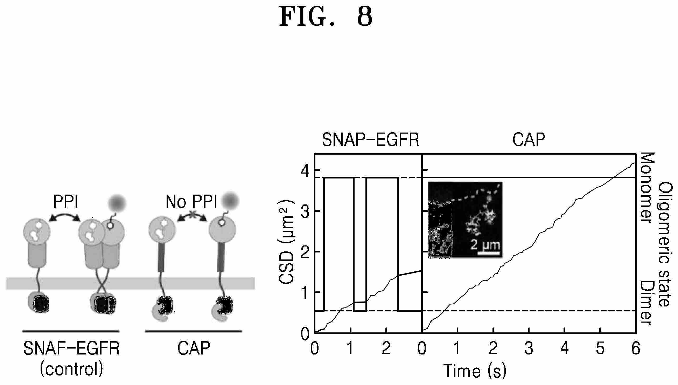

Specifically, single-molecule tracking experiments were performed in living cells, and changes in the diffusivity (D) of individual CAP trajectories were analyzed using a hidden Markov model (HMM). A change in D over time consistent with the Saffman-Delbruck equation indicates interaction with surrounding objects. For comparison, SNAP-tag-fused epidermal growth factor receptor (SNAP-EGFR) was set as a control, with the results shown in FIG. 8.

As shown in FIG. 8, SNAP-EGFR showed a reversible transition between monomeric and dimeric states under similar conditions, whereas CAP and its partial variants (lacking syntenin NTD or linker) showed no detectable interaction with the surrounding environment. This means that within the temporal and spatial resolutions (20 ms, 200 nm), CAP according to an embodiment is not involved in homotypic or heterotypic interactions among its respective components (the coiled-coil domain of TMPDGFR, SNAP, syntenin NTD, or EGFP).

Taken together, these results indicate that CAP according to an embodiment is localized to extracellular vesicles expressing CAP, increases binding efficiency with extracellular vesicles and target functional molecules, and exhibits high stability, including not physically interacting with neighboring proteins.

### Experimental Example 3. Modular Engineering of sEV-CAP

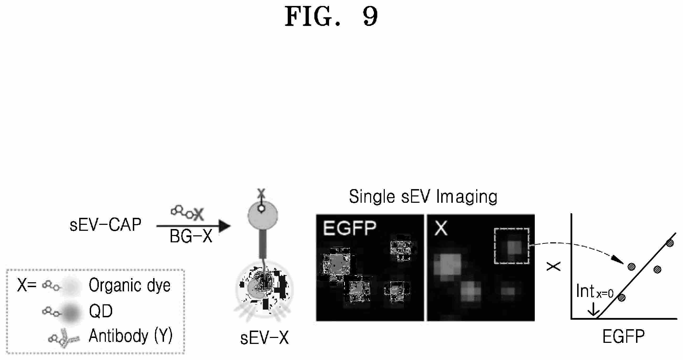

The extracellular vesicle platform expressing chimeric adaptor protein (sEV-CAP) according to an embodiment may be easily functionalized by simply binding to functional molecules conjugated with BG (hereinafter, BG-X). To demonstrate this, experiments were performed on extracellular vesicles (sEV-X) into which various functional molecules (X) were introduced. As described in Example 2, (i) organic molecules represented by Dye, (ii) inorganic materials represented by QD, and (iii) protein therapeutics represented by antibody (cetuximab, hereinafter Y, which is conjugated with R-phycoerythrin (RPE) and modified with BG through BG-GLA-NHS) were introduced. A schematic diagram of the modular engineering of sEV-CAP is shown in FIG. 9.

After incubating the sEV-CAP with each BG-X and removing unbound BG-X, the binding efficiency and presence of each post-synthesis product were evaluated.

### 3.1 Co-localization Analysis

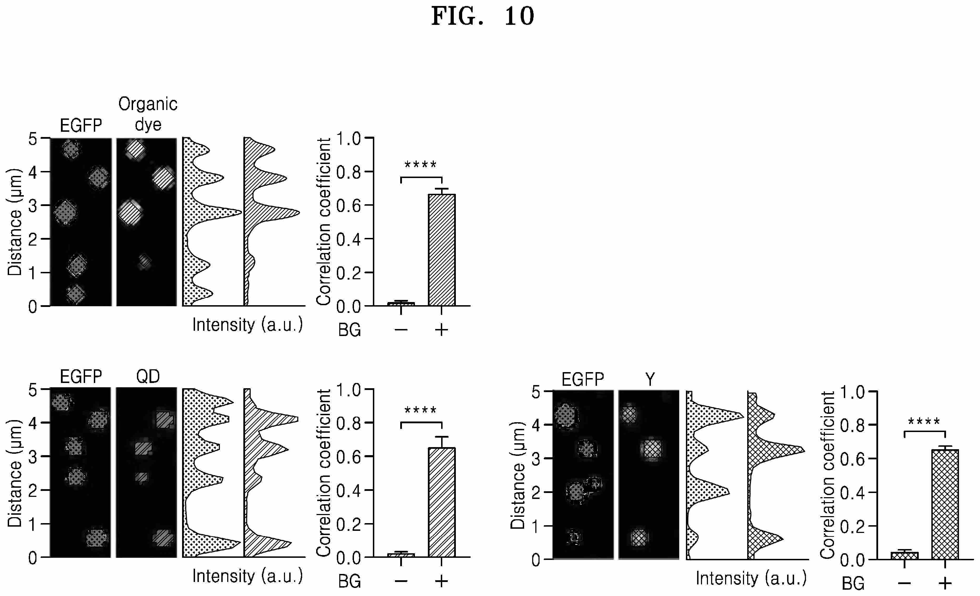

First, co-localization analysis using TIRF microscopy was performed, with the results shown in FIG. 10.

As shown in FIG. 10, high co-localization was observed between the sEV-CAP and all conjugated functional molecules. The Pearson correlation coefficients (r_{(EGFP,X)}) were 0.67 for Dye, 0.65 for QD, and 0.65 for Y, indicating a strong linear relationship.

### 3.2 Calculation of Stoichiometry of CAP and Conjugated Functional Molecules in sEV-X

The stoichiometry of each functional molecule was calculated.

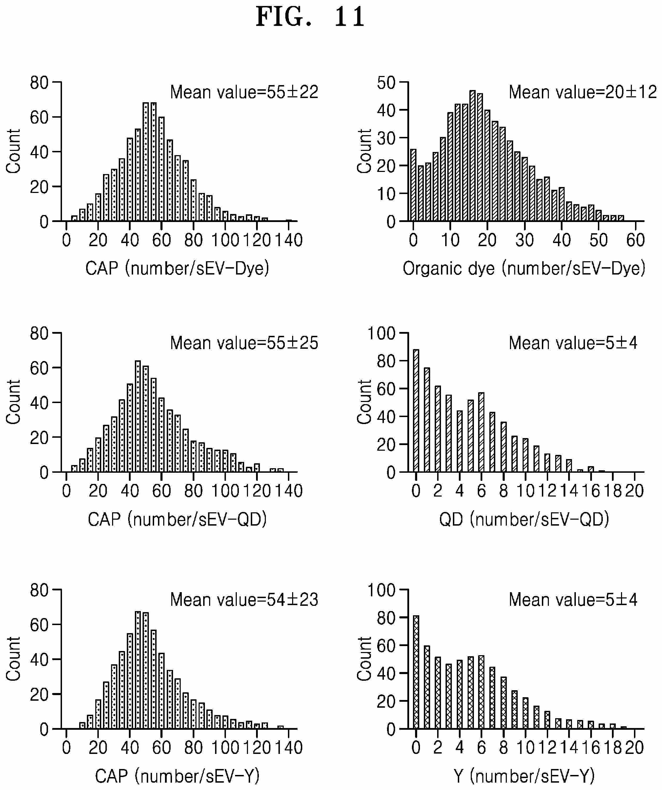

First, to quantify the number of functional molecules in each sEV-X, the fluorescence intensity of a single functional molecule was measured using TIRF. For Dye or QD, single-molecule fluorescence intensity measurements (I_{Dye}, I_{QD}) were obtained. For Y, site-specific click chemistry between the Fc region of cetuximab and fluorescent RPE was used, and the binding stoichiometry ranged from 1:1 to 1:4. It was assumed that the average intensity of RPE in an individual Y molecule represented the intensity of a single Y. Then, stoichiometry of functional molecules was calculated by dividing the fluorescence intensity of functional molecules bound to sEV-CAP, measured using TIRF, by the intensity of each single molecule, with the results shown in FIG. 11.

As shown in FIG. 11, stoichiometry for a single sEV was determined to be 20 ± 12 for Dye, 5 ± 4 for QD, and 5 ± 4 for Y.

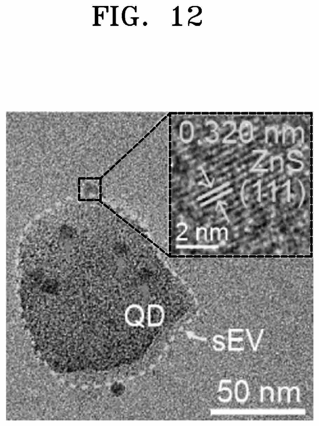

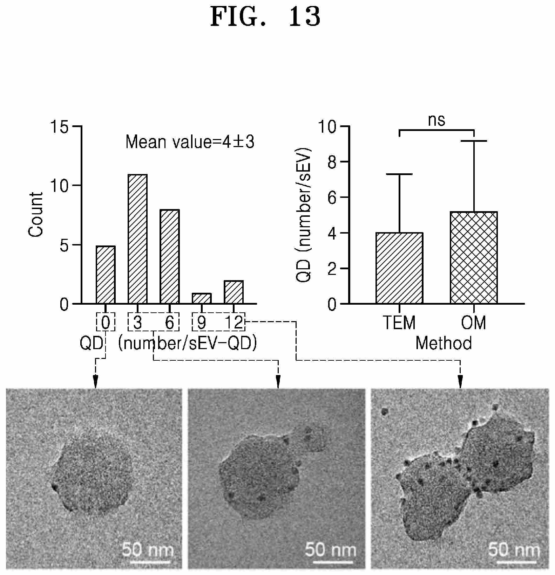

Then, sEV-QD was imaged using TEM. QDs with a CdSe/ZnS core/shell structure appeared darker than carbon-based sEVs, and d-spacing measurement confirmed that the molecules bound to sEVs were QDs, with the results shown in FIG. 12. In addition, the number of QDs bound to a single sEV was quantified through TEM analysis, revealing an average of 4 ± 3 QDs per sEV, with the results shown in FIG. 13.

As shown in FIG. 13, the number of QDs bound to sEVs obtained through TEM analysis showed a similar trend to the optical microscopy-based stoichiometry analysis results of sEV-QD in FIG. 11.

### 3.3 Linear Regression Analysis

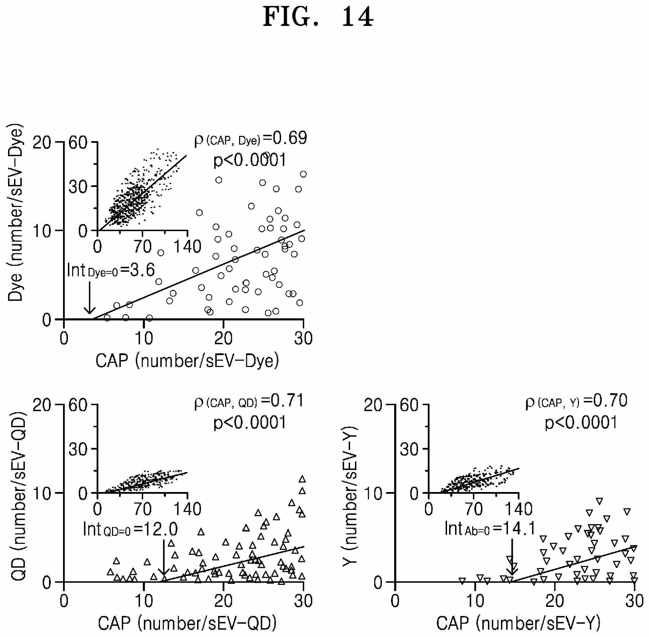

Additionally, linear regression analysis was performed to determine the Pearson correlation between CAP and X in sEV-X, with the results shown in FIG. 14.

As shown in FIG. 14, the correlation coefficients r_{(CAP,X)} were r_{(CAP,Dye)} 0.69 for Dye, r_{(CAP,QD)} 0.71 for QD, and r_{(CAP,Y)} 0.70 for Y, indicating a consistent linear relationship between functionalization reactivity and CAP amount. Furthermore, the minimum amount of CAP required for effective X binding could be estimated through linear regression analysis: 3.6 for Dye (intDye = 0), 12.0 for QD (intQD = 0), and 14.1 for Y (intY = 0).

These results support the validity and modularity of the sEV-CAP functionalization according to an embodiment.

### Experimental Example 4. Evaluation of Therapeutic Effect of sEV-IL2 Conjugate

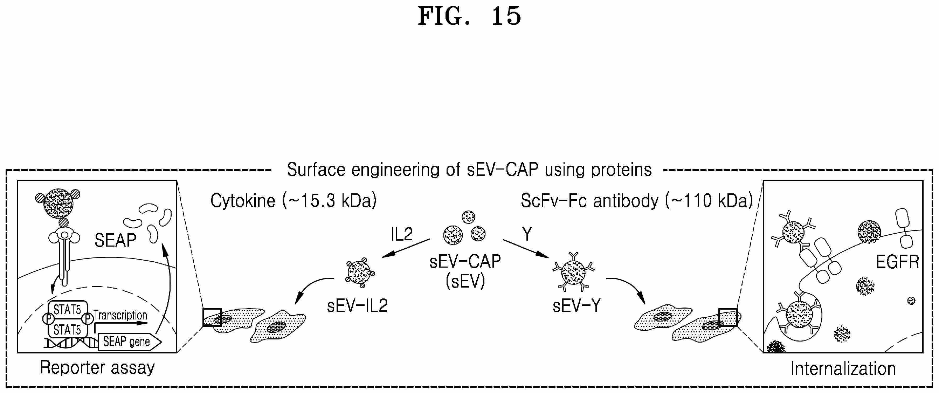

To evaluate the functional improvement of sEVs through the chimeric adaptor protein (CAP) platform, interleukin 2 (IL2; approximately 16 kDa), a low-molecular-weight cytokine with therapeutic potential, was conjugated to sEV-CAP, and the effect of the engineered sEV was evaluated as follows. A schematic diagram of surface engineering and activity testing of sEV-CAP is shown in FIG. 15.

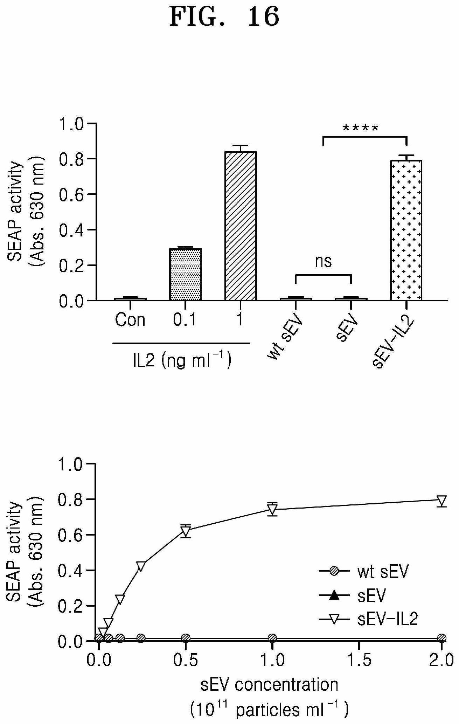

To evaluate signaling mediated by IL2, a reporter system using target cells expressing IL2 receptor (IL2R) was used. Specifically, activity of the secreted embryonic alkaline phosphatase (SEAP) reporter gene induced by IL2R activation was measured, with the results shown in FIG. 16.

As shown in FIG. 16, wt sEVs or sEV-CAP without IL2 did not induce SEAP expression due to the absence of an IL2R-activating molecule, whereas sEV-IL2 induced SEAP expression in a dose-dependent manner. These results indicate that sEV-CAP acquired an additional function of IL2 through surface engineering, and that such an immune-activating agent, when applied to sEV-CAP, may be readily used for the treatment of various immune-related diseases.

### Experimental Example 5. Evaluation of Therapeutic Effect of sEV-Y Conjugate

### 5.1 Confirmation of Cell Penetration and Internalization of sEV-Y Conjugate

To evaluate the functional improvement of sEVs through the chimeric adaptor protein (CAP) platform, cetuximab (ScFv-Fc format; approximately 110 kDa, hereinafter Y), which has a relatively high molecular weight and therapeutic potential, was conjugated to sEV-CAP (sEV-Y), and the effect of the engineered sEV was evaluated as follows. A schematic diagram of surface engineering and activity testing of sEV-CAP is shown in FIG. 15.

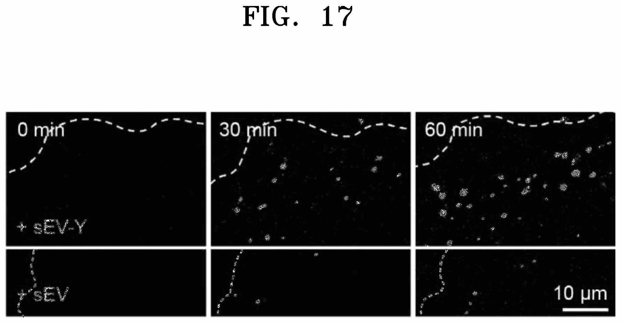

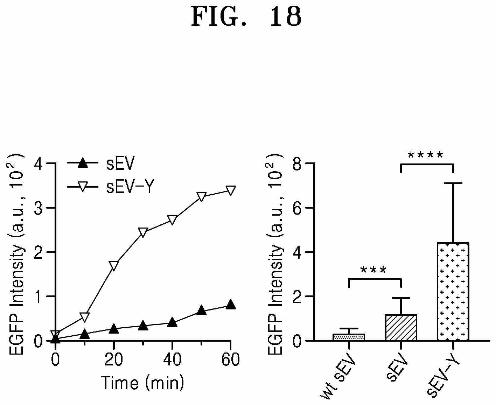

First, cellular uptake efficiency of sEV-Y, which targets EGFR as a specific antigen, in A549 human lung cancer cells was investigated using time-lapse fluorescence imaging and EGFP fluorescence intensity, with the results shown in FIGS. 17 and 18.

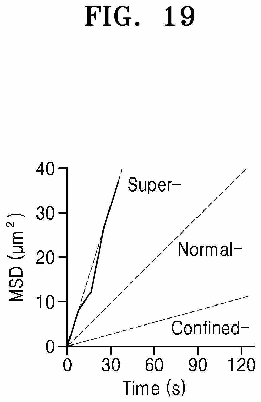

Additionally, representative mean square displacement (MSD) analysis was performed on trajectories showing directional movement 2 hours after treatment. Diffusion types are classified into superdiffusion (active transport), normal diffusion (random movement), and confined diffusion (restricted movement). The results are shown in FIG. 19.

As shown in FIGS. 17 and 18, compared with the sEV-CAP without Y, the amount of sEV-Y on the cell surface increased over time, resulting in approximately 3.8-fold improvement in specific targeting.

As shown in FIG. 19, the sEV-Y was internalized and exhibited superdiffusion indicating active transport along cellular microtubules, which is characteristic of receptor-mediated endocytosis.

That is, these results indicate that the sEV-CAP efficiently achieved cell penetration and internalization using Y conjugated via surface engineering.

### 5.2 Confirmation of Efficacy of sEV-Y as a Drug Carrier

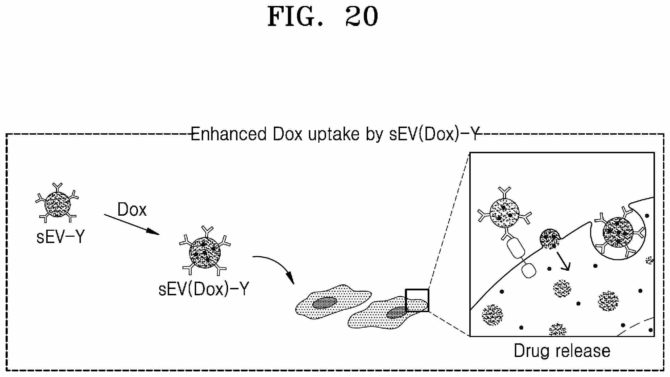

Based on the results of Experimental Example 5.1, the potential of sEV-CAP as a drug carrier was confirmed through the following experiments. FIG. 20 is a schematic diagram showing the drug delivery by the sEV(drug)-Y according to an embodiment.

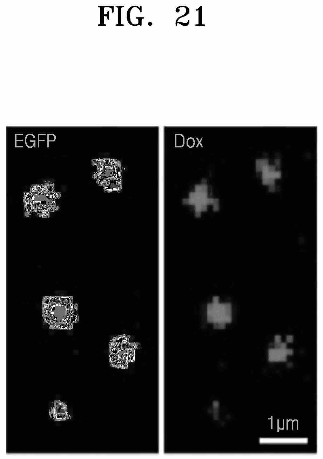

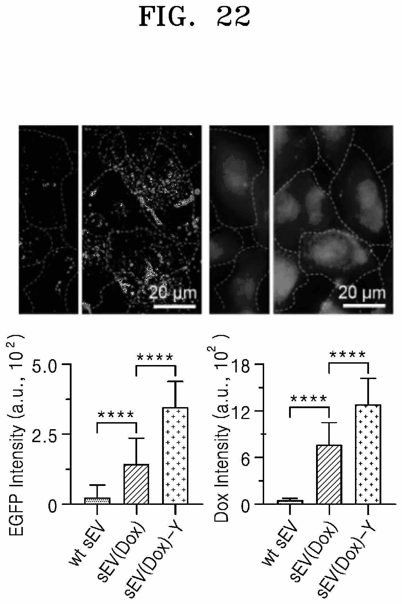

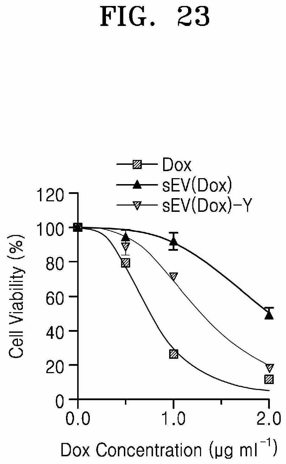

Specifically, doxorubicin (Dox), a chemotherapy drug, was encapsulated in sEV-CAP through electroporation and then conjugated with Y to produce sEV(Dox)-Y. To confirm the internal loading of Dox, the co-localization of EGFP and Dox fluorescence in sEV-CAP was verified through TIRF imaging, with the results shown in FIG. 21. To evaluate whether sEV(Dox)-Y showed improved cellular uptake, EGFP fluorescence images were taken 2 hours after treating A549 cells with sEV(Dox)-Y, and the intensity was quantified, as shown in FIG. 22. In addition, cytotoxicity induced by sEV(Dox)-Y was evaluated 48 hours after treating A549 cells with sEV(Dox)-Y, with the results shown in FIG. 23.

As shown in FIG. 21, the co-localization of EGFP and Dox fluorescence in sEV-CAP was confirmed.

As shown in FIG. 22, the presence of Y in sEV(Dox) improved the drug delivery efficiency to target A549 cells.

As shown in FIG. 23, sEV(Dox)-Y significantly increased cytotoxicity.

These results indicate that the cytotoxicity of sEV(Dox)-Y was attributable to improved drug delivery efficiency resulting from Y-mediated increases in the cellular uptake and internalization of sEV. Furthermore, since no co-localization between EGFP and Dox was observed after internalization, the results suggest that Dox is dispersed within cells via free diffusion, enabling cytosolic drug delivery.

### Experimental Example 6. Confirmation of In Vivo Efficacy of sEV-Y Conjugate

The targeting and drug delivery capabilities of sEVs engineered through the chimeric adaptor protein (CAP) platform were tested in a mouse model bearing subcutaneously implanted tumor cells.

### 6.1 Confirmation of Targeting

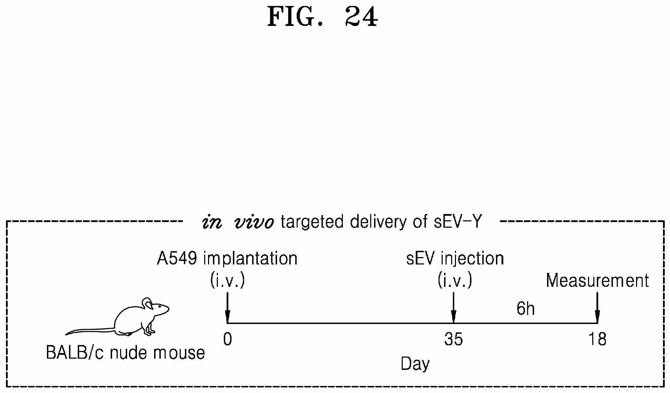

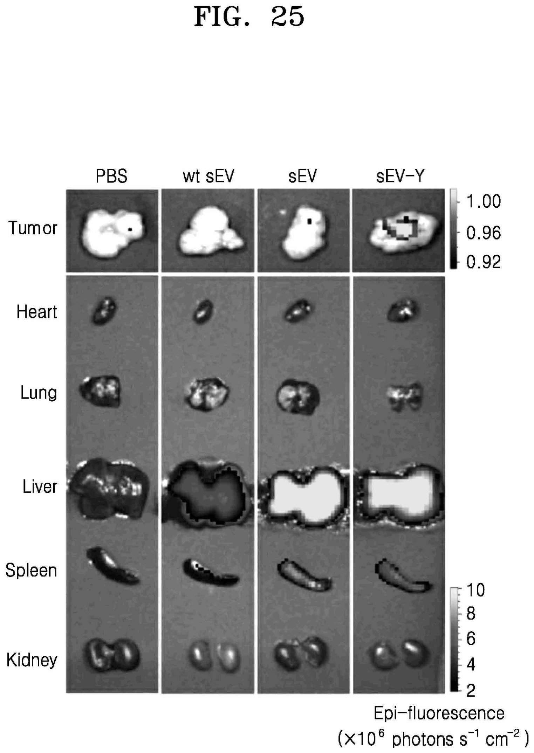

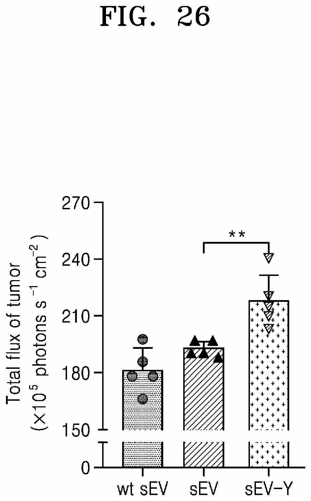

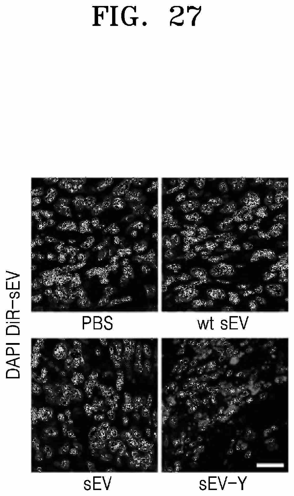

A schematic diagram of the drug administration schedule in mice for the tumor targeting analysis of cetuximab (ScFv-Fc format)-conjugated sEV-CAP and sEV-Y is shown in FIG. 24. Specifically, after administration of wt sEV, sEV, and sEV-Y, fluorescence images were observed in the tumor and major organs, and DiR fluorescence intensity was quantified, with the results shown in FIGS. 25 and 26. Additionally, DiR fluorescence images in frozen tumor sections were observed, as shown in FIG. 27.

As shown in FIGS. 25 to 27, sEV-Y exhibited significant accumulation at the tumor site compared to non-targeted sEVs (wt sEV and sEV-CAP without Y).

### 6.2 Confirmation of Drug Delivery Capability

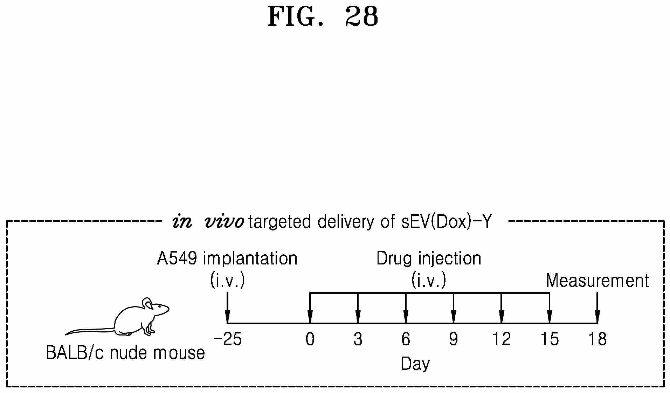

A schematic diagram of the drug administration schedule in mice for a test confirming the drug delivery capability of cetuximab (ScFv-Fc format)-conjugated sEV-CAP and sEV-Y is shown in FIG. 28. Tumor-bearing mice were treated with an equivalent dose (1 mg kg⁻¹) of Dox, administered either in soluble form or encapsulated in non-targeted and targeted sEVs (sEV(Dox) and sEV(Dox)-Y, respectively).

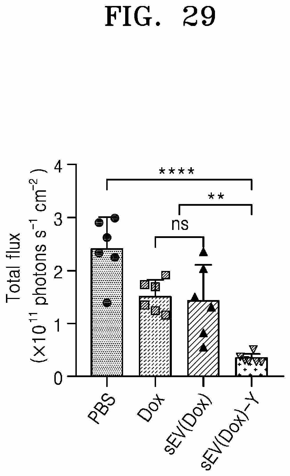

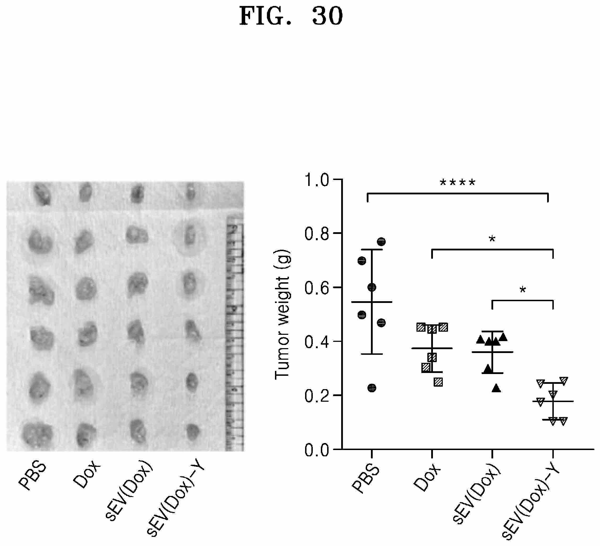

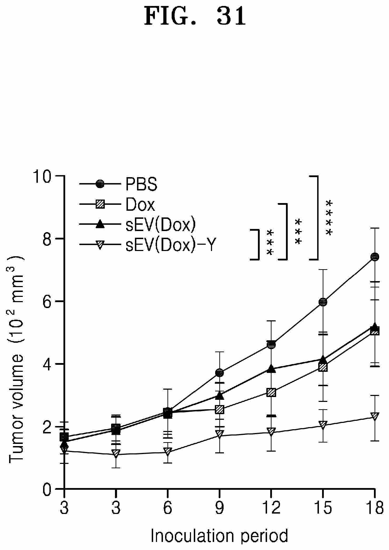

First, tumor bioluminescence was measured on the day of euthanasia after the administration of PBS, Dox, sEV(Dox), or sEV(Dox)-Y, and the quantified results are shown in FIG. 29. Photographs of tumor tissues and the tumor weights obtained on the day of euthanasia are shown in FIG. 30. Tumor volume curves over a period of 19 days from the first day of treatment are shown in FIG. 31.

As shown in FIGS. 29 and 30, soluble Dox and sEV(Dox) reduced tumor bioluminescence by approximately 40 %, whereas sEV(Dox)-Y achieved a significant reduction of 90 % compared to the untreated control group. The tumor volume and weight measured after euthanasia also demonstrated that sEV(Dox)-Y inhibited tumor growth more effectively.

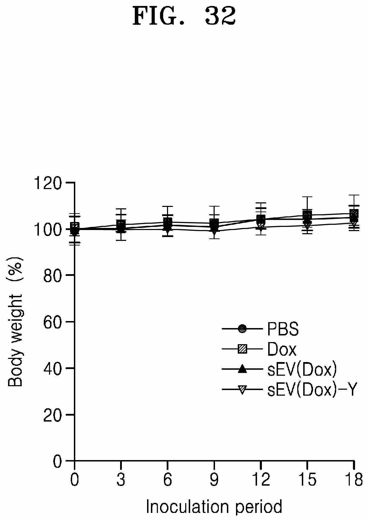

Meanwhile, as shown in FIG. 32, the administered Dox dose of 1 mg kg⁻¹, totaling 6 mg kg⁻¹, was well tolerated, as evidenced by no significant difference in body weight changes between groups. That is, the toxicity associated with the drug delivery system was negligible.

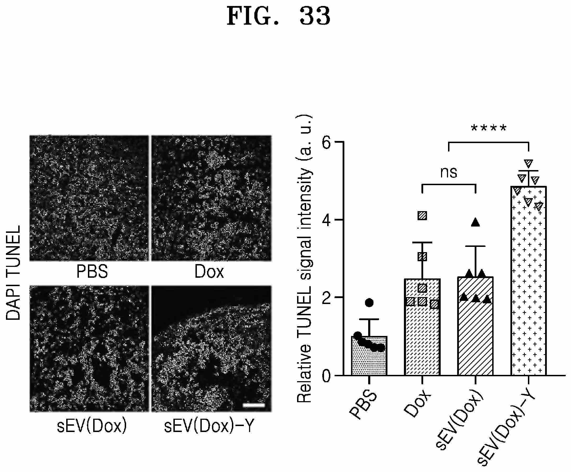

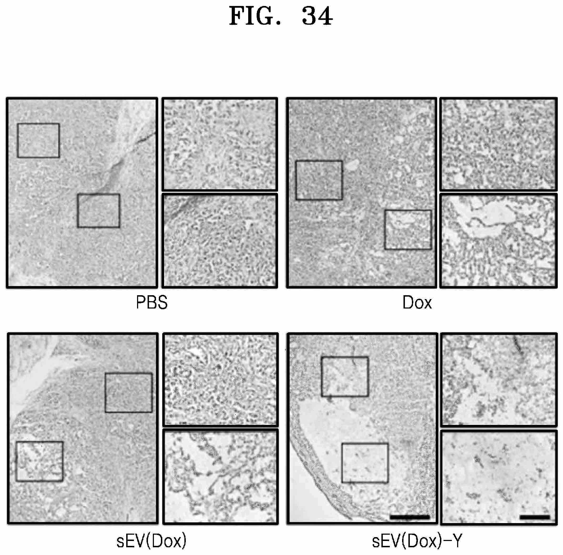

To further evaluate the apoptosis enhanced by sEV(Dox)-Y, terminal deoxynucleotidyl transferase dUTP nick end labeling (TUNEL) analysis was performed, with the results shown in FIG. 33, and histopathological analysis was performed, with the results shown in FIG. 34.

As shown in FIG. 33, the level of GFP bound to fragmented DNA, a marker of apoptosis, increased 2.5-fold upon treatment with Dox and sEV(Dox), but surged 4.8-fold upon treatment with sEV(Dox)-Y compared to the control group. As shown in FIG. 34, this correlated with severe destruction of tissue structure and extensive necrosis within the tumor. This is in contrast to the effects observed when only Dox and sEV(Dox) were administered.

In summary, sEV-CAP is capable of conjugation with functional molecules of various types and sizes. Furthermore, sEV-CAP is effective and safe without cytotoxicity, and may be readily used for the treatment of various tumors when a tumor-targeting therapeutic agent is applied thereto. sEV-CAP has significant potential for clinical translation as a functional technology for developing sEV-based therapeutic agents for various diseases, particularly cancer, depending on the type of agent loaded, and as a versatile biological nanocarrier.

### Reference Examples

### Reference Example 1. Cell Culture

HEK293FT cells were cultured in DMEM containing 10 % FBS and 1 % A/A supplemented with penicillin (100 U ml⁻¹), streptomycin (100 µg ml⁻¹), and amphotericin B (250 ng ml⁻¹). A549-Luc2 cells were cultured in RPMI-1640 medium containing 10 % FBS and 1 % A/A. The cells were cultured at 37 °C under humidified conditions in 5 % CO₂. HEK-Blue^{TM} IL-2 cells (Invivogen) and Expi293F^{TM} cells (Gibco) were cultured according to the manufacturer's instructions.

### Reference Example 2. Optical Microscopy

For total internal reflection fluorescence (TIRF) and epi-fluorescence microscopy, a stereomicroscope (Nikon, ECLPSE Ti2-E) equipped with a perfect focus system (PFS, TI2-N-ND-P), a motorized stage (TI2-S-SE-E), a stage-top incubator capable of temperature and CO₂ control (Okolab, UNO-T-H-PREMIXED), and an electron-multiplying charge-coupled device (EM-CCD, Andor, iXorn Ultra 897) was used. As light sources for the TIRF and Epi microscopy, a laser (Nikon, LU-N4 Laser Unit, 488/561 wavelengths) and a mercury lamp (Nikon, C-HGFIE Intensilight) were used, respectively. Imaging was performed using a 100× objective lens (Nikon, 1.49 N.A., oil-immersion, CFI SR HP Apochromat TIRF). DAPI, DiR, and TUNEL signals of tissue sections were imaged using a confocal microscope (Olympus, FV1200) equipped with 405 nm, 488 nm, and 635 nm lasers. Histological images of tissues were imaged using a fluorescence microscope (Nikon, Eclipse Ni-E) equipped with 4× and 20× objective lenses. All imaging data from optical microscopy were processed and analyzed using ImageJ software.

Regarding samples for microscopy experiments, in the case of cells, the cells were seeded and stabilized for 24 hours on a fibronectin-coated glass-bottom dish (MatTek). When transfection was required, the cells were transfected with a vector and further cultured for 24 hours. When a designated label was used, the cells were incubated with the label at 37 °C for 15 minutes and then washed three times with PBS to remove unbound labels. In the case of sEVs or functional molecules (dyes, QDs, antibodies), the sEVs or each of the molecules were immobilized on a glass-bottom dish for 15 minutes. Then, the dish was washed three times with PBS to remove excess materials.

### Reference Example 3. Quantitative Analysis of EGFP Expression at Single-Cell and Single sEV Levels.

Stable cell lines and sEVs expressing an EGFP-fused engineering protein (CAP) were imaged using a TIRF microscope with a 488 nm laser. Snapshots were taken with an exposure time of 100 ms. The number of EGFPs in a single sEV was quantified based on the single fluorescence intensity of EGFP.

### Reference Example 4. Evaluation of Expression Level and Binding Efficiency for BG on Cell Membrane

Stable cell lines expressing individual SNAP-tag constructs (CAP) were observed using a total internal reflection fluorescence (TIRF) microscope with a 488 nm laser to evaluate the expression level. To evaluate the binding efficiency for BG, the same stable cell lines were incubated with 1.0 µM SNAP 549 or 1.0 nM streptavidin-QD. In the case of cells treated with the streptavidin-QD, the cells were pre-labeled with 1.0 µM BG-biotin and then washed three times with PBS. Then, the cells were imaged at the same position using the TIRF microscope to detect the SNAP 549 or the streptavidin-QD using a 561 nm laser and to detect EGFP using a 488 nm laser. The expression level of each construct was evaluated through fluorescence intensity measurement of EGFP. The binding efficiency was quantified as a ratio of the SNAP 549 or QD fluorescence intensity to the EGFP fluorescence intensity. All imaging was performed with an exposure time of 100 ms.

### Reference Example 5. Single-Molecule Tracking

Stable cell lines (cells expressing CAP) and HEK293FT cells transfected with a vector encoding a SNAP-tag-fused epidermal growth factor receptor (SNAP-EGFR; control) were treated with 1.0 µM BG-biotin and then incubated with 1.0 nM streptavidin-QD. Fluorescence trajectories of each QD were obtained using a TIRF microscope at 561 nm. Time-lapse imaging was performed for 3 minutes without intervals at an exposure of 20 ms per frame.

### Reference Example 6. Analysis of Size and Concentration of sEVs

Size distribution and the absolute number of sEVs were analyzed through Nanoparticle Tracking Analysis (NTA; Malvern Panalytical, NanoSight LM10). The sEVs were diluted in PBS until there were approximately 50 to 150 particles per frame. The sEVs were captured for 30 seconds at a rate of 30 frames s⁻¹ using NTA software version 2.3 (NanoSight). The production yield of the sEVs was calculated by dividing the total number of sEVs harvested from the culture supernatant by the number of seeded cells.

### Reference Example 7. Western blot Analysis

The purified sEVs or cells were lysed and quantified using a micro BCA protein assay kit. The lysates were boiled at 95 °C for 5 minutes, and then a sample buffer was added thereto. Each sample (15 µg) was loaded onto a Bolt 4-12 % Bis-Tris plus gel (Invitrogen) operating at 100 V and transferred to a nitrocellulose membrane via an iBlot system (Invitrogen). The membrane was cut to enable blotting for various antibodies. After blocking with 5 % bovine serum albumin in Tris-buffered saline containing 0.05 % Tween 20 (TBST), each membrane was incubated with a primary antibody at 4 °C overnight. After washing with TBST, the blot was stained with a secondary antibody and imaged using a ChemiDoc imaging system (Bio-Rad, ChemicDox XRS+ system).

### Reference Example 8. Quantitative Analysis of CAP and Conjugated Functional Molecules (X) in a Single sEV-X

CAP (EGFP) and each molecule (streptavidin-QD, SNAP 549, and Cet-RPE) were immobilized on a dish for microscopic observation. For QD, SNAP 549, and Cet-RPE, 100 µl of a 10.0 pM solution in PBS buffer was incubated for 15 minutes. EGFP was prepared by immobilizing sEV-CAP for 15 minutes.

Fluorescence trajectories of QD, SNAP 549, and EGFP were obtained using a TIRF microscope, wherein a 561 nm laser was used for QD and SNAP 549, and a 488 nm laser was used for EGFP. Time-lapse imaging was performed for 3 minutes without intervals at an exposure of 100 ms per frame. Main peaks corresponding to a single QD, SNAP 549, or EGFP molecule were identified by constructing fluorescence intensity histograms from the collected trajectories. Cet-RPE was imaged using the TIRF microscope with a 561 nm laser and snapshots were taken at an exposure of 100 ms, and the average intensity of a single Cet-RPE was determined by analyzing a fluorescence intensity histogram. Functional molecules in a single sEV-X were quantified using the average fluorescence intensity values of the individual QD, SNAP 549, Cet-RPE, and EGFP. To ensure accurate fluorescence analysis of EGFP in a single sEV-CAP, QD and Cet-RPE were imaged at 488 nm and 561 nm lasers. The fluorescence intensity under the 488 nm laser was quantified relative to the 561 nm laser, and this value was excluded from subsequent quantification experiments using EGFP.

After functionalizing the sEV-CAP, the EGFP was imaged using a 488 nm laser, and the conjugated molecule X was imaged using a 561 nm laser in the TIRF microscope. The fluorescence intensity of individual EGFP spots (sEV-X) was measured, and the corresponding fluorescence intensity of X was measured at the same position. The total fluorescence intensity of EGFP and X in a single sEV-X was divided by the intensity of each single molecule for quantitative analysis.

### Reference Example 9. Linear Regression Analysis of CAP with Functional Molecules (X) in a Single sEV-X

The numbers of CAP and X in a single sEV-X were obtained from the quantitative analysis in Reference Example 8. Linear regression analysis was performed to display a best-fit line and derive an X-intercept. Additionally, 95 % confidence intervals were calculated for both X and Y intercepts to evaluate the correlation between CAP and functional molecules.

### Reference Example 10. Pearson Correlation of CAP and Functional Molecules (X) in a Single sEV-X

The Pearson correlation coefficient (r) was calculated to evaluate the linear correlation between the fluorescence intensities of CAP (EGFP) and X or between the numbers of CAP and X. This coefficient quantifies the degree of a linear relationship between two variables from -1 (perfect negative correlation) to 1 (perfect positive correlation), where 0 indicates no linear relationship. In the co-localization analysis, x and y represent the fluorescence intensities of EGFP and X, respectively. In the quantitative analysis for a single sEV-X, x and y represent the numbers of CAP and X molecules, respectively. The correlation was calculated using the following equation.$r = \frac{\sum \left({x}_{i}-\overline{x}\right) \left({y}_{i}-\overline{y}\right)}{\sqrt{\sum {\left({x}_{i}-\overline{x}\right)}^{2} \sum {\left({y}_{i}-\overline{y}\right)}^{2}}}$

Here, xᵢ and yᵢ represent individual sample points, and x̅ and y̅ are the means of the x and y samples, respectively.

### Reference Example 11. sEV and Dox Cellular Uptake Assay

A549-Luc2 cells were seeded in a fibronectin-coated dish at a density of 5 × 10³ cells per well. After 24 hours, the cells were incubated with different treatments according to the experimental focus. To prepare cetuximab-conjugated sEVs, cetuximab was first reacted with a 20-fold molar excess of BG-GLA-NHS for 30 minutes at room temperature. Then, the mixture was subjected to a 7 K MWCO Zeba spin desalting column to remove unconjugated BG-GLA-NHS. The sEVs were then reacted with a 100-fold molar excess of BG-conjugated cetuximab (BG-Cet) at room temperature overnight. For sEV uptake experiments, the cells were treated with the sEVs (3.7 × 10⁹ particles ml⁻¹). To analyze Dox cellular uptake by sEVs, the cells were treated with Dox (1 µg ml⁻¹), sEV(Dox) (1.1 × 10¹⁰ particles ml⁻¹), or sEV(Dox)-Y (0.9 × 10¹⁰ particles ml⁻¹), which corresponds to an equivalent dose of Dox.

The green fluorescence of the sEVs and the red fluorescence of the Dox were observed using an epi-fluorescence microscope. For sEV delivery experiments, time-lapse imaging was performed at 10-minute intervals for 1 hour after treatment. Thereafter, the cells were washed with 1 ml of PBS to remove extracellular sEVs, and the sEVs internalized into the cells were imaged to quantify delivery efficiency. In addition, fluorescence trajectories of individual sEVs in the cells were obtained. Time-lapse imaging was performed continuously for 3 minutes without intervals at an exposure of 100 ms per frame. For Dox delivery experiments, the cells were washed with 1 ml of PBS 2 hours after treatment to remove extracellular sEVs and Dox, and the internalized sEVs and Dox were imaged. The dish was placed in a preheated microscope stage-top incubator maintained at 37 °C, 5 % CO₂, and 80 % humidity during imaging.

### Reference Example 12. Reporter Cell Assay

A reporter assay using HEK-Blue^{TM} IL2 cells was performed according to the manufacturer's instructions. The cells were seeded in a 96-well plate at a density of 5 × 10⁴ cells per well and cultured with IL2 or sEVs. After 24 hours, 20 µl of the supernatant per well was incubated with 180 µl of Quanti-Blue^{™} solution under humidified conditions (37 °C, 5 % CO₂). After 1 hour, secreted alkaline phosphatase was quantified by measuring absorbance at 630 nm.

### Reference Example 13. MTS Assay

A549-Luc2 cells were seeded in a 96-well plate at a density of 1 × 10⁴ cells per well. After 24 hours, the cells were cultured with Dox or sEVs loaded with Dox. Cell viability was analyzed using a CellTiter 96^{®} AQueous one solution reagent. After adding 20 µl of the reagent, the microplate was incubated under humidified conditions (37 °C, 5 % CO₂) for 1 hour, and then absorbance was measured at 490 nm.

### Reference Example 14. Animal Experiments

All animal experiments were performed in accordance with the animal experiment guidelines approved by the Institutional Animal Care and Use Committee (IACUC) of DGIST (approval number 23032701-0000). Male BALB/c-nu mice (6 to 8 weeks old, Orient Bio) were used for the study. A549-Luc2 cells (1 × 10⁷ cells in 100 µl of PBS) were mixed with an equal volume of Matrigel (Corning) and then subcutaneously injected into the mice. Tumor volume (V) was calculated as V = length × width² / 2.

To analyze the biodistribution of sEVs, the mice were randomly grouped when the tumor volume reached approximately 600 mm³. PBS or DiR-labeled sEVs (2 × 10¹¹ particles per mouse) were intravenously injected via the tail vein. The mice were euthanized 4 hours after injection, and major organs (tumor, heart, lungs, liver, spleen, and kidneys) were collected to quantify fluorescence signals using an in vivo imaging system (Perkin Elmer, IVIS Spectrum). To evaluate the therapeutic efficacy of Dox-loaded sEVs, the mice were randomly divided into four groups when the tumor volume reached approximately 150 to 170 mm³, and an equivalent dose of Dox (1 mg kg⁻¹), either in soluble form or encapsulated in sEVs, was intravenously injected six times at three-day intervals. Before euthanasia, bioluminescence signals were detected using an in vivo imaging system, and tumor tissue was excised, weighed, and subjected to histological analysis.

### Reference Example 15. Immunofluorescence and Histological Analysis

The tissues harvested from mice in Reference Example 14 were fixed in 4 % paraformaldehyde, dehydrated with 30 % sucrose in PBS, and then embedded in OCT compound. The frozen tissues were sectioned at a thickness of 10 µm, and the slides were washed with PBS. Apoptosis was detected using a TUNEL assay kit following the manufacturer's instructions. Nuclei were stained with DAPI for 5 minutes at room temperature. After washing with PBS, the samples were mounted using an antifade mounting medium. For hematoxylin and eosin staining, the slides were stained with hematoxylin for 3 minutes and washed with sterile distilled water. Subsequently, the slides were stained with eosin for 1 minute and washed with water. After dehydration in 95 % and absolute ethanol, the samples were mounted using a mounting medium.

### Reference Example 16. Statistical Analysis

All data are expressed as the mean ± standard deviation of the indicated replicates. Statistical analysis was performed using GraphPad Prism 8.0 software with a two-tailed Student's t-test and one-way ANOVA with a post-hoc test. Quantitative and linear regression analyses and calculation of the Pearson correlation coefficient for the numbers of CAP and X in a single sEV-X were performed using GraphPad Prism 8.0. ImageJ software was used for the calculation of the Pearson correlation coefficient for the fluorescence intensities of CAP (EGFP) and X, as well as for the measurement of sEV diameters and TUNEL signals. Data for the Pearson correlation coefficient were analyzed from ten individual images. Data for the TUNEL signal of each mouse were expressed as the mean of five alternate sections.

### Reference Example 17. Experimental Materials

### 1) Materials for cell culture and transfection

HEK293FT (Thermo Fisher), Expi293F^{TM} (Gibco), HEK-Blue^{TM} IL-2 (Invivogen), A549-Luc2 (ATCC), fetal bovine serum (FBS; Hyclone), Dulbecco's modified eagle medium (DMEM; Hyclone), Roswell Park Memorial Institute (RPMI) 1640 (Hyclone), Expi293F^{™} expression medium (Gibco), antibiotic-antimycotic solution (A/A; GenDEPOT), insulin-transferrin-selenium (Gibco), hygromycin B (Gibco), puromycin (Invivogen), normocin (Invivogen), HEK-Blue^{™} CLR selection (Invivogen), Lipofectamine^{®} 2000 (Invitrogen), and ExpiFectamine^{™} 293 transfection kit (Gibco).

### 2) Materials for general experimentation

SNAP-surface 549 (SNAP 549; BioLabs), QD 605 streptavidin conjugate (streptavidin-QD; Thermo Fisher), SNAP-biotin^{®} (BG-biotin; BioLabs), phosphate-buffered saline (PBS; pH 7.4, Biowest), fibronectin from bovine plasma (fibronectin; Sigma-Aldrich), and ethanol (99 %, Alfa Aesar).

### 3) Materials for surface modification of sEV-CAP

Recombinant human interleukin 2 (IL2; Genscript), streptavidin gold nanoparticle conjugate (streptavidin-Au NP; abcam), Texas Red-(ACTG)₅-NH₂ (Cosmogentech), BG-GLA-NHS (NEB), and dimethyl sulfoxide (DMSO; 99 %, Sigma-Aldrich).

### 4) Materials for in vitro experimentation

Cloning restriction enzymes (BsmB I, EcoR I, Sfi I; Enzynomics), T4 ligase (NEB), XL1-blue electro-competent E. coli (Agilent), carbenicillin (Invitrogen), Zeocin (Gibco), sodium phosphate monobasic dihydrate (≥ 99 %; Sigma-Aldrich), sodium phosphate dibasic dihydrate (≥ 98.5 %; Sigma-Aldrich), glycine (> 99 %; TCI America), hydrochloric acid (HCl; aqueous solution, 37 %, Sigma-Aldrich), tris(hydroxymethyl)aminomethane (Tris; ≥ 99.8 %, Sigma-Aldrich), doxorubicin (Dox; Biosynth), CellTiter 96^{®} AQueous one solution reagent (Promega), Quanti-BlueTM solution (Invivogen), electroporation buffer (Invitrogen), NeonTM resuspension buffer R (Invitrogen), formalin solution (10 %, neutral buffered; Sigma-Aldrich), glutaraldehyde (50 % in H2O, Sigma-Aldrich), sodium cacodylate trihydrate (SC; 98 %, Sigma-Aldrich), SNAP-tag^{®} purified protein (NEB), 5X SDS-PAGE loading buffer (Biosesang), coomassie brilliant blue R 250 (Sigma-Aldrich), and methanol (99 %, Alfa-Aesar).

### 5) Antibodies

Anti-SNAP-tag polyclonal rabbit antibody (NEB, P9310), anti-GM130 monoclonal mouse antibody (BD Biosciences, 610822), anti-β-actin polyclonal rabbit antibody (Cell Signaling, 4967), anti-ALIX monoclonal mouse antibody (Abcam, ab117600), anti-Hsp70 monoclonal mouse antibody (Abcam, ab2787), anti-TSG101 polyclonal rabbit antibody (Abcam, ab30871), anti-CD9 polyclonal rabbit antibody (Abcam, ab223052), HRP-conjugated anti-mouse IgG antibody (Cell Signaling, 7076), and HRP-conjugated anti-rabbit IgG antibody (Cell Signaling, 7074).

### 6) Materials for in vivo experimentation

1,1'-Dioctadecyl-3,3,3',3'-tetramethylindotricarbocyanine iodide (DiR; Invitrogen), Matrigel^{®} basement membrane matrix (Corning), FSC22 frozen section compound (Leica biosystem), paraformaldehyde (4 %, Biosesang), terminal deoxynucleotidyl transferase dUTP nick end labeling (TUNEL) assay kit 488 nm (Cell Signaling), 4',6-diamidino-2-phenylindole, dihydrochloride (DAPI; Thermo Scientific), hematoxylin (Dako), eosin (Dako), ProLongTM gold antifade mounting reagent (Invitrogen), Eukitt^{®} quick-hardening mounting reagent (Sigma-Aldrich).

The foregoing description is illustrative of the present disclosure, and it will be apparent to those skilled in the art that various changes and modifications may be made without departing from the spirit and scope of the disclosure. Accordingly, the embodiments disclosed herein are intended to be illustrative rather than limiting, and the scope of the disclosure is defined by the appended claims rather than by the foregoing description.

## Claims

1. A chimeric adaptor protein, comprising: an O6-benzylguanine-binding protein; a transmembrane domain; and a signaling domain.

2. The chimeric adaptor protein of claim 1, wherein the O6-benzylguanine-binding protein comprises a self-labeling protein (SLP).

3. The chimeric adaptor protein of claim 1, wherein the O6-benzylguanine-binding protein comprises a SNAP-tag.

4. The chimeric adaptor protein of claim 1, wherein the transmembrane domain comprises one or more transmembrane domains selected from the group consisting of platelet-derived growth factor receptor (PDGFR), epidermal growth factor receptor (EGFR), fibroblast growth factor receptor (FGFR), vascular endothelial growth factor receptor (VEGFR), hepatocyte growth factor receptor (HGFR), tropomyosin receptor kinase (Trk), insulin receptor (IR), leukocyte receptor tyrosine kinase (LTK), angiopoietin receptor, cholecystokinin (CCK) receptor, neurotrophic factor (NGF) receptor, receptor tyrosine kinase-like orphan receptors (ROR), discoidin domain receptor (DDR), rearranged during transfection receptor (RETR), tyrosine-protein kinase-like (PTK), related to receptor tyrosine kinase (RYK), muscle-specific kinase (MuSK), CD63, CD9, and CD81.

5. The chimeric adaptor protein of claim 1, wherein the signaling domain comprises one or more signaling domains selected from the group consisting of CD9, CD63, a C-terminus of CD63, CD81, LAMP-1, LAMP-2, syntaxin-3, syntenin-1, syntenin-2, syndecan-1, syndecan-4, and prostaglandin F2 receptor negative regulator.

6. The chimeric adaptor protein of claim 1, wherein the signaling domain comprises an N-terminal sorting domain of syntenin.

7. The chimeric adaptor protein of claim 1, wherein the O6-benzylguanine-binding protein and the transmembrane domain are linked directly or via a linker.

8. The chimeric adaptor protein of claim 7, wherein the linker comprises a rigid linker.

9. The chimeric adaptor protein of claim 1, further comprising a reporter.

10. The chimeric adaptor protein of claim 1, comprising an amino acid sequence set forth in SEQ ID NO: 1.

11. A construct, comprising a gene encoding the chimeric adaptor protein of any one of claims 1 to 10.

12. A vector, comprising the construct of claim 11.

13. A recombinant cell, comprising the construct of claim 11, or a vector comprising the construct.

14. An extracellular vesicle, comprising the chimeric adaptor protein of any one of claims 1 to 10, or derived from the recombinant cell of claim 13.

15. The extracellular vesicle of claim 14, wherein the chimeric adaptor protein is further linked to an O6-benzylguanine, or to an O6-benzylguanine and a functional molecule.

16. The extracellular vesicle of claim 15, wherein the functional molecule is an organic fluorophore, an inorganic material, an antibody or an antigen-binding fragment thereof, or a cytokine.

17. The extracellular vesicle of claim 14, wherein a drug is loaded in the extracellular vesicle.
